(19) Europäisches Patentamt
European Patent Office
Office européen des brevets

(11) Veröffentlichungsnummer : **0 174 459 B1**

(12) # EUROPÄISCHE PATENTSCHRIFT

(45) Veröffentlichungstag der Patentschrift : **03.01.90**

(51) Int. Cl.$^5$ : **C 07 D461/00**, A 61 K 31/475

(21) Anmeldenummer : **85108624.9**

(22) Anmeldetag : **11.07.85**

(54) **9- beziehungsweise 11-(Nitro)-apovincaminsäurederivate, Verfahren zu ihrer Herstellung und diese Verbindungen enthaltende Arzneimittel.**

(30) Priorität : **11.07.84 HU 270484**

(43) Veröffentlichungstag der Anmeldung : **19.03.86 Patentblatt 86/12**

(45) Bekanntmachung des Hinweises auf die Patenterteilung : **03.01.90 Patentblatt 90/01**

(84) Benannte Vertragsstaaten : **AT BE CH DE FR GB IT LI NL SE**

(56) Entgegenhaltungen : **FR-A- 2 342 980**

(73) Patentinhaber : **RICHTER GEDEON VEGYESZETI GYAR R.T.**
**Gyömröi ut 19-21**
**H-1475 Budapest X (HU)**

(72) Erfinder : **Vedres, András, Dr. Diplomchem.**
**Füredi u. 56**
**H-1144 Budapest (HU)**
Erfinder : **Szántay, Csaba, Dr.**
**Zsombolyai u. 8**
**H-1113 Budapest (HU)**
Erfinder : **Moldvai, István**
**Kossuth L. u. 107**
**H-1212 Budapest (HU)**
Erfinder : **Stefkò, Béla, Dr. Diplomchem.**
**Orlay u. 2/b**
**H-1117 Budapest (HU)**
Erfinder : **Groò, Dòra, Dr.**
**Naprafoprgò u. 27**
**H-1021 Budapest (HU)**
Erfinder : **Kárpáti, Egon, Dr.**
**Mihályfi E. u. 7/b**
**H-1022 Budapest (HU)**
Erfinder : **Kiss, Béla**
**OTP ltp. A/1**
**H-2220 Vecsés (HU)**
Erfinder : **Pálosi, Eva, Dr.**
**Vend u. 21**
**H-1025 Budapest (HU)**
Erfinder : **Riesz, Miklòs, Dr.**
**Damjanich u. 52**
**H-1071 Budapest (HU)**
Erfinder : **Szombathelyi, Zsolt, Dr.**
**Munkacsy M. u. 12**
**H-1063 Budapest (HU)**

**EP 0 174 459 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Erfinder : **Szporny, Lászlò, Dr.**
**Szabolcska M. u. 7**
**H-1114 Budapest (HU)**
Erfinder : **Zájer, Mária, Dipl.-Chem.**
**Zalka M. tér 1**
**H-1102 Budapest (HU)**

(74) Vertreter : **Beszédes, Stephan G., Dr. Patentanwalt**
**Münchener Strasse 80a Postfach 1168**
**D-8060 Dachau (DE)**

**Beschreibung**

Die Erfindung betrifft neue 9- beziehungsweise 11-(Nitro)-apovincaminsäurederivate, ein chemisch eigenartiges Verfahren zur Herstellung derselben und von bekannten 9- beziehungsweise 11-(Nitro)-apovincaminsäurederivaten und diese neuen und/oder bekannten Verbindungen enthaltende Arzneimittel, insbesondere mit gefäßerweiternder, spasmolytischer, antihypoxischer und antikonvulsiver Wirkung.

Von den Nitroapovincaminsäurederivaten sind bisher nur 9- und 11-(Nitro)-apovincamin, auch 9- und 11-(Nitro)-apovincaminsäuremethylester genannt, aus der französischen Offenlegungsschrift 2 342 980 bekanntgeworden, von einer pharmakologischen Wirkung dieser Verbindungen ist jedoch keine Rede, vielmehr dienen sie nur als Zwischenprodukte. Gemäß dieser Druckschrift werden diese Verbindungen auf zweierlei Weise hergestellt. Beim einen Reaktionsweg wird Apovincamin direkt nitriert, wobei ein Gemisch der Nitroisomere anfällt. Aus diesem wird das 9-(Nitro)-apovincamin durch Kristallisieren aus Methanol abgetrennt; das 11-(Nitro)-apovincamin bleibt in der methanolischen Mutterlauge und kann aus dieser chromatographisch abgetrennt werden, jedoch nur als amorphe Substanz. Gemäß dem zweiten Reaktionsweg werden das 9- beziehungsweise 11-(Nitro)-vincamin durch Dehydratisieren beziehungsweise Wasserentzug hergestellt. Als dehydratisierendes beziehungsweise wasserentziehendes Mittel wird wasserfreie Ameisensäure verwendet, die Reaktionstemperatur beträgt 100 °C, und die Reaktionszeiten — 48 beziehungsweise 24 Stunden — sind recht lang. Zur Reinigung der Endprodukte sind gegebenenfalls ebenfalls chromatographische Verfahren erforderlich.

Der Erfindung liegt die Aufgabe zugrunde, überlegene pharmakologische Wirkungen aufweisende neue 9- beziehungsweise 11-(Nitro)-apovincaminsäurederivate, ein einfaches und elastisches Verfahren zur Herstellung derselben und von bekannten 9- beziehungsweise 11-(Nitro)-apovincaminsäurederivaten und diese Verbindungen enthaltende Arzneimittel zu schaffen.

Das Obige wurde überraschenderweise durch die Erfindung erreicht.

Gegenstand der Erfindung sind 9- beziehungsweise 11-(Nitro)-apovincaminsäurederivate der allgemeinen Formel

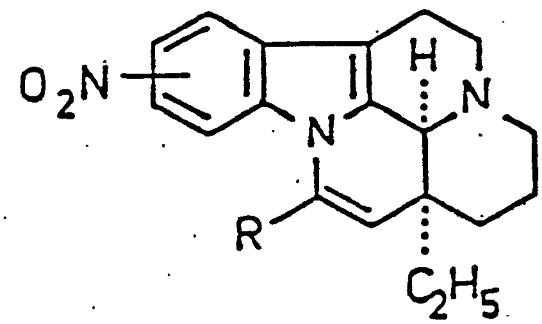

I

worin R für einen Halogencarbonylrest der allgemeinen Formel

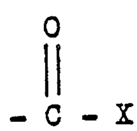

II

in welchletzterer X ein Halogenatom bedeutet, oder einen Kohlenwasserstoffoxycarbonylrest der allgemeinen Formel

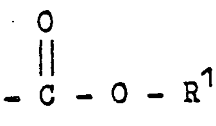

III

in welchletzterer $R^1$ einen aliphatischen Kohlenwasserstoffrest mit 1 bis 10 Kohlenstoffatom(en), cycloaliphatischen Kohlenwasserstoffrest mit 3 bis 8 Kohlenstoffatomen oder aromatischen Kohlenwasserstoffrest mit 6 bis 14 Kohlenstoffatomen bedeutet,

wobei ein derartiger Rest bzw. derartige Reste durch [ein] Halogenatom(e), [eine] primäre, sekundäre und/oder tertiäre Aminogruppe(n), [eine] Hydroxygruppe(n), [einen] Arylrest(e) mit 6 bis 14 Kohlenstoffatomen, [ein] Alkyl-, Alkoxy- und/oder Alkylthiorest(e) mit [je] 1 bis 8 Kohlenstoffatom(en), [ein] Trifluormethylrest(e), [eine] Carboxylgruppe(n), [einen] Alkoxycarbonylrest(e) mit 1 bis 8 Kohlenstoffamton(en) im Alkylteil, [eine] Thio-, Sulfinyl-, Sulfonyl-, Nitro-, Keto-, Aldehyd- und/oder Cyangruppe(n) und/oder [einen] 1 oder mehr gleiche oder verschiedene Heteroatom(e) aufweisende[n] Heteroarylrest(e) sowie gegebenenfalls zusätzlich zu diesen Gliedern mit 4 zur Bildung eines Benzolrings ankondensierten Gliedern, der bzw. die gegebenenfalls durch 1 oder mehr der aufgeführten Substituenten substituiert sein

kann bzw. können, substituiert sein kann [können], oder einen Aminocarbonylrest der allgemeinen Formel

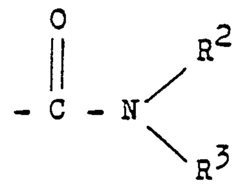

IV

in welchletzterer

$R^2$ und $R^3$ unabhängig voneinander Wasserstoff beziehungsweise Alkylreste mit 1 bis 8 Kohlenstoffatom(en), welche gegebenenfalls 2 miteinander verbunden als Alkylenrest zusammen mit dem Stickstoffatom, an welches sie gebunden sind, einen Pyrrolidino-, Piperidino-, Imidazolidino-, Piperazino- oder Morpholinorest darstellen, bedeuten, oder

$R^2$ Wasserstoff bedeutet und

$R^3$ eine Aminogruppe darstellt,

oder eine Cyanogruppe steht und die Nitrogruppe in der 9- oder 11-Stellung ist,

mit der weiteren Maßgabe, daß

R von einem Kohlenwasserstoffoxycarbonylrest der allgemeinen Formel III, bei welchem $R^1$ einen nicht substituierten Methylrest bedeutet, verschieden ist,

sowie ihre optisch aktiven Isomere und Salze. Die erfindungsgemäßen 9- beziehungsweise 11-(Nitro)-apovincaminsäurederivate umfassen also sowohl racemische als auch optisch aktive, von welchletzterem die rechtsdrehenden bevorzugt sind.

In der allgemeinen Formel II kann X für jedes beliebige Halogenatom, wie für ein Fluor-, Chlor-, Brom- oder Jodatom, stehen. Vorzugsweise ist das Halogenatom, für welches X steht, ein Chlor- oder Bromatom, insbesondere Chloratom.

Der aliphatische Kohlenwasserstoffrest, für welchen $R^1$ stehen kann, kann geradkettig oder verzweigt, gesättigt oder ungesättigt sein. Beispiele sind der Methyl-, Äthyl-, n-Propyl-, Isopropyl-, n-Butyl-, sek.-Butyl-, tert.-Butyl-, Isobutyl-, n-Pentyl-, Isopentyl-, n-Hexyl-, Isohexyl-, n-Heptyl-, Isoheptyl-, n-Octyl-, Isooctyl-, Vinyl-, Acryl-, Methacryl- und Propenylrest. Der aliphatische Kohlenwasserstoffrest, für welchen $R^1$ stehen kann, kann ein Alkylrest, insbesondere ein solcher mit 2 bis 8, ganz besonders 2 bis 4, vor allem 2 oder 3, Kohlenstoffatomen, oder ein Alkenyl- oder Alkinylrest, insbesondere ein solcher mit 2 bis 4, ganz besonders 2 oder 3, Kohlenstoffatomen, vor allem der Allylrest oder der Propargylrest, sein.

Beispiele für den cycloaliphatischen Kohlenwasserstoffrest, für welchen $R^1$ stehen kann, sind der Cyclopropyl-, Cyclobutyl-, Cyclopentyl-, Cyclohexyl-, Cycloheptyl- und Cyclooctylrest. Vorzugsweise ist der cycloaliphatische Kohlenwasserstoffrest, für welchen $R^1$ stehen kann, ein Cycloalkylrest, insbesondere ein solcher mit 5 bis 7, ganz besonders 5 oder 6, vor allem 5, Kohlenstoffatomen.

Der aromatische Kohlenwasserstoffrest, für welchen $R^1$ stehen kann, kann monocyclisch, nämlich der Phenylrest, oder polycyclisch nicht kondensiert [isoliert], insbesondere der Diphenylrest, oder polycyclisch kondensiert, insbesondere ein Naphthylrest, sein.

Vorzugsweise ist beziehungsweise sind der beziehungsweise die Substituent(en), durch welche[n] der aliphatische, cycloaliphatische beziehungsweise aromatische Kohlenwasserstoffrest, für den $R^1$ steht, substituiert sein kann, soweit es sich um die folgenden handelt, wie folgt: Von den Aminogruppen [eine] Dialkylaminogruppe(n) mit 1 bis 10, insbesondere 1 bis 4, vor allem 1, Kohlenstoffatom(en) in jedem Alkylteil, von den Arylresten [ein] Phenyl-, Diphenyl- oder Naphthylrest(e), von den Alkyl-, Alkoxy- bzw. Alkylthioresten, [ein] Alkyl-, Alkoxy- und/oder Alkylthiorest(e) mit [je] 1 bis 4, ganz besonders 1 oder 2, Kohlenstoffatom(en), von den Alkoxycarboxylresten [ein] Alkoxycarbonylrest(e) mit 1 bis 4, ganz besonders 1 oder 2, vor allem 1, Kohlenstoffatom(en) im Alkylteil, von den Heteroarylresten [ein] Stickstoff, Sauerstoff und/oder Schwefel, aufweisende[r] Heteroarylrest(e), insbesondere 5- bis 7-gliedrige[r], ganz besonders 5- oder 6-gliedrige[r], vor allem [ein] Pyrrolyl-, Furyl-, Thienyl-, Pyridyl-, Pyranyl-, Pyrazolyl-, Imidazolyl-, Pyrimidinyl-, Indolyl- und/oder Chinolylrest(e). Im Falle, daß $R^1$ für einen cycloaliphatischen Rest steht, hat dieser als Substituenten besonders bevorzugt 1 oder mehr Alkylrest(e).

Der beziehungsweise die Alkylrest(e), für welche[n] $R^2$ und/oder $R^3$ stehen kann beziehungsweise können, kann beziehungsweise können der beziehungsweise die obigen sein.

Vorzugsweise ist beziehungsweise sind der beziehungsweise die Alkylrest(e), für welche[n] $R^2$ und/oder $R^3$ stehen kann beziehungsweise können, [ein] solche[r] mit 1 bis 4, insbesondere 1 oder 2, Kohlenstoffatom(en).

Die Salze der 9- beziehungsweise 11-(Nitro)-apovincaminsäurederivate der allgemeinen Formel I können Säureadditionssalze oder quaternäre Salze sein. Die Säureadditionssalze können zum Beispiel solche mit den folgenden Säuren sein: Mit anorganischen Säuren, zum Beispiel Salzsäure, Bromwasserstoffsäure, Schwefelsäure, Phosphorsäure, Überhalogensäuren, wie Überchlorsäure, und mit organischen Säuren, zum Beispiel Ameisensäure, Essigsäure, Propionsäure, Glykolsäure, Maleinsäure, Hydroxymaleinsäure, Fumarsäure, Bernsteinsäure, Weinsäure, Ascorbinsäure, Citronensäure, Apfelsäure, Salicylsäure, Milchsäure, Zimtsäure, Benzoesäure, Phenylessigsäure, p-Aminobenzoesäure, p-Hydroxybenzoesäure, p-Aminosalicylsäure, Alkylsulfonsäuren, wie Methansulfonsäure beziehungsweise Äthansulfonsäure, cycloaliphatischen Sulfonsäuren, wie Cyclohexylsulfonsäure, Arylsulfonsäuren, wie Naphthalinsulfonsäure, p-Toluolsulfonsäure beziehungsweise Sulfanilsäure, und schließlich Aminosäuren wie Asparaginsäure, Glutaminsäure, N-Acetylasparaginsäure beziehungsweise N-Acetylglutarsäure. Als quaternäre Salze kommen zum Beispiel Methojodide, Methobromide, Methosulfate beziehungsweise deren Alkylhomologen mit 2 bis 4 Kohlenstoffatomen, zum Beispiel Äthojodide, in Frage.

Besonders bevorzugte erfindungsgemäße 9- beziehungsweise 11-(Nitro)-apovincaminsäurederivate sind 9-(Nitro)-apovincaminsäure-äthylester, 11-(Nitro)-apovincaminsäure-äthylester, 9-(Nitro)-apovincaminsäurechlorid, 11-(Nitro)-apovincaminsäurechlorid, 11-[Nitro]-apovincaminsäure-{[(dimethylamino)-n-propyl]-ester}, insbesondere 11-[Nitro]-apovincaminsäure-[3'-(dimethylamino)-n-propyl]-ester, 9-[Nitro]-apovincaminsäure-{[(dimethylamino)-n-propyl]-ester}, insbesondere 9-[Nitro]-apovincaminsäure-[3'-(dimethylamino)-n-propyl]-ester, 11-(Nitro)-apovincaminsäure-benzylester, 9-(Nitro)-apovincaminsäure-benzylester, 11-(Nitro)-apovincaminsäure-pyridylmethylester, insbesondere 11-(Nitro)-apovincaminsäure-{[(3'-(pyridyl)-methyl]-ester}, 9-(Nitro)-apovincaminsäure-pyridylmethylester, insbesondere 9-(Nitro)-apovincaminsäure-{[(3'-(pyridyl)-methyl]-ester}, 9-(Nitro)-apovincaminsäure-(hydroxyäthylester), insbesondere 9-(Nitro)-apovincaminsäure-{[2'-(hydroxy)-äthyl]-ester}, 11-(Nitro)-apovincaminsäure-(hydroxyäthylester), insbesondere 11-(Nitro)-apovincaminsäure-{[2'-(hydroxy)-äthyl]-ester}, 9-(Nitro)-apovincaminsäure-isobutylester, 11-(Nitro)-apovincaminsäure-isobutylester, 9-(Nitro)-apovincaminsäure-n-octylester, 11-(Nitro)-apovincaminsäure-n-octylester, 9-(Nitro)-apovincaminsäure-n-propylester, 11-(Nitro)-apovincaminsäure-n-propylester, 9-(Nitro)-apovincaminsäurenitril, 11-(Nitro)-apovincaminsäurenitril, 9-(Nitro)-apovincaminsäureamid, 11-(Nitro)-apovincaminsäureamid, 9-(Nitro)-apovincaminsäure-diäthylamid, 11-(Nitro)-apovincaminsäure-diäthylamid, 9-[Nitro]-apovincaminsäure-[4'-(methyl)-piperidid], 11-[Nitro]-apovincaminsäure-[4'-(methyl)-piperidid], 11-(Nitro)-apovincaminsäure-phenylester, 9-(Nitro)-apovincaminsäure-phenylester, 11-(Nitro)-apovincaminsäure-hydrazid, 9-(Nitro)-apovincaminsäure-hydrazid, 9-(Nitro)-apovincaminsäure-propargylester, 11-(Nitro)-apovincaminsäure-propargylester, 9-[Nitro]-apovincaminsäure-{[2'-tri-(fluor)-äthyl]-ester}, 11-[Nitro]-apovincaminsäure-{[2'-tri-(fluor)-äthyl]-ester}, 9-(Nitro)-apovincaminsäure-allylester, 11-(Nitro)-apovincaminsäure-allylester, 9-(Nitro)-apovincaminsäure-cyclopentylester und 11-(Nitro)-apovincaminsäure-cyclopentylester sowie ihre optisch aktiven Isomere und Hydrochloride.

Gegenstand der Erfindung ist auch ein Verfahren zur Herstellung der erfindungsgemäßen 9- beziehungsweise 11-(Nitro)-apovincaminsäurederivate beziehungsweise von 9- beziehungsweise 11-(Nitro)-apovincamin, welches dadurch gekennzeichnet ist, daß

a) racemische oder optisch aktive 9- beziehungsweise 11-(Nitro)-apovincaminsäuren beziehungsweise Derivate derselben der allgemeinen Formel

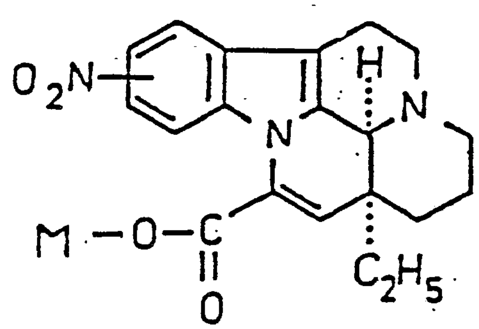

V

worin M für Wasserstoff, ein Metallatom oder eine Ammoniumgruppe steht, oder falls M von einem Metallatom verschieden ist, auch deren Säureadditionssalze beziehungsweise quaternäre Salze mit Hydroxyverbindungen beziehungsweise Estern der allgemeinen Formel

$$R^1 - Y \qquad VI,$$

worin

$R^1$ die oben angegebenen Bedeutungen hat oder einen nicht substituierten Methylrest darstellt

und

Y für eine Hydroxygruppe oder einen Säurerest steht,

gegebenenfalls in Gegenwart von Basen, oder mit Halogenierungsmitteln umgesetzt werden und gegebenenfalls die erhaltenen 9- beziehungsweise 11-(Nitro)-apovincaminsäurederivate der allgemeinen Formel I, bei welchen R für einen Halogencarbonylrest der allgemeinen Formel II steht, mit nukleophilen Reagenzien der allgemeinen Formeln

$$H - O - R^1 \qquad VII$$

beziehungsweise

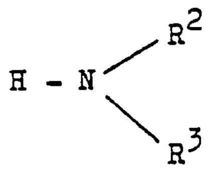

VIII

worin $R^1$, $R^2$ und $R^3$ die oben angegebenen Bedeutungen haben oder $R^1$ für einen nicht substituierten Methylrest steht, umgesetzt werden und gegebenenfalls die erhaltenen 9- beziehungsweise 11-(Nitro)-apovincaminsäurederivate der allgemeinen Formel I, bei welchen R für einen Aminocarbonylrest der allgemeinen Formel IV, bei welchem $R^2$ und $R^3$ Wasserstoffatome bedeuten, steht, mit wasserentziehenden Mitteln dehydratisiert werden oder

b) racemische oder optisch aktive Apovincaminsäurederivate der allgemeinen Formel

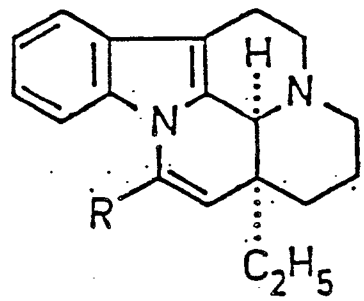

IX

worin R die oben angegebenen Bedeutungen hat, oder deren Säureadditionssalze nitriert werden, sowie gegebenenfalls die erhaltenen 9- beziehungsweise 11-(Nitro)-apovincaminsäurederivate der allgemeinen Formel I, bei welchen R für einen Kohlenwasserstoffoxycarbonylrest der allgemeinen Formel III, bei welchem $R^1$ einen Alkylrest mit 1 oder 2 Kohlenstoffatom(en) bedeutet, steht, beziehungsweise ihre Säureadditionssalze umgeestert oder amidiert werden und/oder gegebenenfalls die erhaltenen 9- beziehungsweise 11-(Nitro)-apovincaminsäurederivate der allgemeinen Formel I in Säureadditionssalze oder quaternäre Salze überführt werden und/oder gegebenenfalls die erhaltenen Salze der 9- beziehungsweise 11-(Nitro)-apovincaminsäurederivate der allgemeinen Formel I in die freien 9- beziehungsweise 11-(Nitro)-apovincaminsäurederivate der allgemeinen Formel I oder in andere Säureadditionssalze beziehungsweise quaternäre Salze überführt werden und/oder gegebenenfalls eine Spaltung der erhaltenen racemischen 9- beziehungsweise 11-(Nitro)-apovincaminsäurederivate der allgemeinen Formel I beziehungsweise von deren Säureadditionssalzen beziehungsweise quaternären Salzen beziehungsweise eine Racemisierung der entsprechenden optisch aktiven Verbindungen in Stereoisomere vorgenommen wird.

Bei den bei der obigen Variante a) des erfindungsgemäßen Verfahrens als Ausgangssubstanzen eingesetzten 9- beziehungsweise 11-(Nitro)-apovincaminsäurederivaten der allgemeinen Formel V kann M für ein beliebiges Metallatom stehen, wobei die Elemente der I. und II. Hauptgruppe, das heißt Alkalimetalle, wie Natrium, Kalium und Lithium, oder Erdalkalimetalle, wie Magnesium und Calcium, sowie ferner Edelmetalle, wie Silber, bevorzugt sind. Als Säureanion Y kommen vor allem Säurereste beliebiger anorganischer Säuren, zum Beispiel Fluoride, Chloride, Bromide, Jodide und Sulfate, in Frage. Soweit es sich um Säurereste organischer Säuren handelt, sind darunter Acyloxyreste zu verstehen.

Das erfindungsgemäße Verfahren ermöglicht nicht nur die Herstellung zahlreicher, höchst unterschiedlicher 9- beziehungsweise 11-(Nitro)-apovincaminsäurederivate, sondern hat darüberhinaus gegenüber demjenigen bekannten Verfahren, welches von 9- beziehungsweise 11-(Nitro)-vincamin ausgeht, zahlreiche Vorteile.

Diese Vorteile sind wie folgt. Das erfindungsgemäße Verfahren ist äußerst elastisch, denn mit ihm können die unterschiedlichen Säurederivate der allgemeinen Formel I, die Säurehalogenide, Ester, Amide, Hydrazide und Nitrile, aus den Ausgangsverbindungen der allgemeinen Formeln V beziehungsweise IX und den entsprechenden Reaktionspartnern immer unter optimalen Bedingungen, die dem physikalischen und chemischen Charakter der betreffenden Verbindung entsprechend gewählt werden, hergestellt werden. Das erfindungsgemäße Verfahren hat den weiteren Vorteil, daß die Doppelbindung zwischen den Kohlenstoffatomen 14 und 15 nicht an den die Nitrogruppe aufweisenden Derivaten erfolgt wie gemäß dem genannten Verfahren der genannten Druckschrift, da die Ausgangsverbindungen der allgemeinen Formeln V und IX von vornherein diese Doppelbindung haben. Gemäß der genannten Druckschrift kann diese Doppelbindung zwar an Vincaminderivaten durch Dehydratisieren beziehungsweise Wasserentzug ausgebildet werden, dieser Vorgang erfordert jedoch im Falle von Nitroderivaten energischere Reaktionsbedingungen, weswegen die Gefahr der Bildung von Nebenprodukten größer ist, wodurch einerseits das Endprodukt verunreinigt und andererseits auch die Ausbeute am Endprodukt vermindert wird. Ein weiterer Vorteil des erfindungsgemäßen Verfahrens besteht darin, daß die 9- und 11-(Nitro)-apovincaminsäurederivate in derjenigen Verfahrensphase voneinander getrennt werden können, in welcher dies am vorteilhaftesten ist, zum Beispiel in einem früheren Abschnitt des Verfahrens und sogar auch noch bei der Herstellung der Ausgangsstoffe. Eigene Reproduktionsversuche in Zusammenhang mit der oben erörterten französischen Offenlegungsschrift 2 342 980 ergaben, daß neben den erwähnten beiden Isomeren, dem 9- und dem 11-(Nitro)-apovincamin, noch in einer Menge von etwa 30 % ein Nebenprodukt entsteht und eben dieses Nebenprodukt für die Schwierigkeiten bei der Abtrennung des in der Mutterlauge verbliebenen 11-(Nitro)-apovincamines verantwortlich ist. Das erfindungsgemäße Verfahren hat schließlich den Vorteil, daß jede seiner Varianten in sehr hoher Ausbeute ein leicht abtrennbares und außerordentlich reines Endprodukt ergibt.

Die im erfindungsgemäßen Verfahren als Ausgangsstoffe einsetzbaren 9- beziehungsweise 11-(Nitro)-apovincaminsäuren beziehungsweise Derivate derselben der allgemeinen Formel V sind neue Verbindungen, die und deren Herstellung Gegenstand der europäischen Offenlegungsschriften 173 824 und 170 926 ist.

Das Wesen der Herstellung der 9- beziehungsweise 11-(Nitro)-apovincaminsäuren beziehungsweise Derivate derselben der allgemeinen Formel V besteht darin, daß Apovincaminsäure nitriert wird und die erhaltenen 9- und 11-(Nitro)-apovincaminsäure in sehr einfacher Weise voneinander getrennt werden.

Die im erfindungsgemäßen Verfahren als Ausgangsverbindungen einsetzbaren Apovincaminsäurederivate der allgemeinen Formel IX können zum Beispiel durch Halogenieren, Verestern oder Amidieren der Apovincaminsäure hergestellt worden sein.

Bei der Variante a) des erfindungsgemäßen Verfahrens können als Ausgangsstoffe außer den 9- beziehungsweise 11-(Nitro)-apovincaminsäuren der allgemeinen Formel V, bei welchen M für Wasserstoff steht, auch deren Salze verwendet werden. Diese Salze können Säureadditionssalze oder Metall- beziehungsweise Ammoniumsalze sein. Letztere sind mit einer anorganischen Base, zum Beispiel einem Alkalihydroxyd, wie Natrium- oder Kaliumhydroxyd, oder mit Ammoniumhydroxyd hergestellt worden. Schließlich kommen auch quaternäre Salze, die zum Beispiel mit Alkylhalogeniden mit 1 bis 4 Kohlenstoffatom(en), wie Methyl-, Äthyl- oder n-Propylchlorid, Methyl-, Äthyl- oder n-Propylbromid oder Methyl-, Äthyl- oder n-Propyljodid oder mit Alkylsulfaten mit 1 bis 4 Kohlenstoffatom(en) im beziehungsweise in jedem Alkylteil, zum Beispiel Dimethyl- oder Diäthylsulfat, hergestellt worden sein können, in Frage.

Setzt man im Verfahren a) 9- oder 11-Nitroapovincaminsäure (M = H) ein, so steht in der Verbindung $R^1$—Y Y bevorzugt für eine Hydroxylgruppe, d. h. es handelt sich um eine Reaktion zwischen einer Säure und einem Alkohol, um eine direkte Veresterung. Diese Umsetzung wird, vorzugsweise in einem Überschuß des Alkohols $R^1$—Y, unter Ausschluß der Luftfeuchtigkeit und vorzugsweise in Gegenwart eines Katalysators ausgeführt. Als Katalysator sind anorganische und organische Säuren, zum Beispiel Salzsäure — zweckmäßig in Gasform -, und ferner Schwefelsäure und p-Toluolsäure geeignet. Es kann in Gegenwart eines wasserentziehenden Mittels, zum Beispiel eines geeignet gewählten Molekularsiebes, gearbeitet werden. Diese Arbeitsweise ist besonders bei der Herstellung von Verbindungen der allgemeinen Formel I mit R = Formel III, worin $R^1$ für einen Alkylrest mit 1 bis 4 Kohlenstoffatom(en) steht, geeignet.

Steht im Verfahren a) der Rest Y in der Verbindung $R^1$—Y für einen Säurerest, so kann M in der Ausgangsverbindung V sowohl für Wasserstoff wie auch für Metallatom oder Ammoniumgruppe stehen. Für den Fall M = H führt man das Verfahren zweckmäßig in Gegenwart einer anorganischen Base, zum Beispiel eines Alkalihydroxyds (Lithium-, Kalium-, Natriumhydroxyd) oder Alkalicarbonats (Natrium-, Kaliumcarbonat) oder in Gegenwart von Ammoniumhydroxyd aus. Steht in der allgemeinen Formel V M für Wasserstoff, so wird im ersten Schritt der Reaktion eigentlich in situ das entsprechende Metallsalz oder Ammoniumsalz gebildet, und anschließend reagiert das Carboxylatanion mit der Verbindung $R^1$—Y.

Steht in der Verbindung $R^1$—Y der Säurerest Y für ein Halogenidion, d. h. ist das Reagens zum Beispiel ein Alkylhalogenid, so wird die Umsetzung zweckmäßig in einem organischen aprotischen dipolaren Lösungsmittel, zum Beispiel in Acetonitril oder Dimethylformamid, vorgenommen. Steht in der Verbindung V M für Metallatom oder Ammoniumgruppe, so wird das Metall- beziehungsweise Ammoniumsalz in dem Lösungsmittel suspendiert und das entsprechende Alkylhalogenid in geringem Überschuß zu der Suspension gegeben. Die Reaktionstemperatur beträgt 50-120 °C. Auf diese Weise werden bevorzugt Verbindungen der allgemeinen Formel I hergestellt, in denen R = Formel III mit $R^1 = C_{1-4}$-Alkylrest ist. Als Lösungsmittel können auch Gemische organischer Lösungsmittel mit Wasser verwendet werden, in diesem Fall wird in Gegenwart eines zweckmäßig gewählten Phasentransfer-Katalysators, zum Beispiel in Gegenwart eines Tetraalkylammoniumhalogenids, gearbeitet.

Steht in der Verbindung $R^1$—Y das Säureanion Y für Sulfat, so kann das Reagens zum Beispiel ein Dialkylsulfat sein. Die Umsetzung der Verbindung I mit einem Dialkylsulfat ist eine in der Alkaloidchemie und insbesondere für Vincaminderivate bisher wenig angewendete Veresterungs- beziehungsweise Alkylierungsmethode. Der große Vorteil dieser Reaktion besteht darin, daß sie selektiv, ohne Quaternisierungsreaktionen in hoher Ausbeute zu dem gewünschten Produkt führt. Die Verfahrensweise ist besonders vorteilhaft bei der Herstellung von Verbindungen der allgemeinen Formel I, in denen R = Formel III mit $R^1 = C_{1-4}$-Alkylrest ist. Die Umsetzung wird zweckmäßig in Gegenwart einer anorganischen Base, zum Beispiel eines Alkalihydroxyds (wie Lithium-, Kalium-, Natriumhydroxyd), eines Alkalicarbonats (wie Kalium-, Natriumcarbonat) oder in Gegenwart von Ammoniumhydroxyd, mit einem

geringen Überschuß des Dialkylsulfates, vorteilhaft bei Raumtemperatur, ausgeführt. Als Lösungsmittel ist Wasser bevorzugt, jedoch können auch inerte organische Lösungsmittel Verwendung finden.

Im erfindungsgemäßen Verfahren a) können die Verbindungen der allgemeinen Formel I mit jedem beliebigen Halogenierungsmittel umgesetzt werden, das geeignet ist, die Carbonsäuregruppe zum Carbonsäurehalogenid umzuwandeln. Geeignet sind zum Beispiel die Thionylhalogenide, wie Thionylchlorid und Thionylbromid. Als Lösungsmittel dient zweckmäßig der Überschuß des Halogenierungsmittels, zum Beispiel des Thionylchlorids, jedoch kann die Reaktion auch in einem inerten aprotischen organischen Lösungsmittel ausgeführt werden. Geeignete Lösungsmittel sind unter anderem die aromatischen Kohlenwasserstoffe, zum Beispiel Benzol, oder aliphatische Halogenkohlenwasserstoffe, wie Chloroform. Die Halogenierungsreaktion kann innerhalb eines breiten Temperaturbereiches ausgeführt werden, im allgemeinen arbeitet man bei 0-100 °C. Nach Beendigung der Halogenierung fallen die Verbindungen der allgemeinen Formel I, in denen R = Formel II mit X = Halogenatom ist, in Form ihrer Hydrohalogenide an. Diese Hydrohalogenide werden dann gewünschtenfalls mit den nucleophilen Reagenzien der allgemeinen Formeln XII beziehungsweise VIII umgesetzt. Diese nukleophilen Reagenzien können zum Beispiel gegebenenfalls substituierte aliphatische oder aromatische Alkohole (wie Methanol, Äthanol und Benzylalkohol und ferner Pyridylcarbinol und Dimethylaminopropanol, oder Ammoniak, primäre oder sekundäre Amine, zum Beispiel Diäthylamin, Benzylamin, Piperidin oder Morpholin, sein. Die Umsetzung wird in einem inerten organischen Lösungsmittel vorgenommen. Geeignete Lösungsmittel sind Äther, Halogenkohlenwasserstoffe, wie Chloroform oder aromatische Kohlenwasserstoffe, wie Benzol. Als Lösungsmittel kann jedoch auch der Überschuß des nukleophilen Reagens dienen. Die Reaktionstemperatur kann innerhalb eines breiten Bereiches variiert werden, vorzugsweise arbeitet man bei 0-150 °C. Gegebenenfalls — vor allem, wenn das nukleophile Reagens ein Alkohol der allgemeinen Formel VII ist — arbeitet man in Gegenwart eines Saurebindemittels. Als Säurebindemittel kommen organische oder anorganische Basen, zum Beispiel Triäthylamin, Pyridin oder geglühte Alkalicarbonate, wie Kalium- oder Natriumcarbonat, in Frage. Ist das nukleophile Reagens ein primäres oder sekundäres oder aromatisches oder alicyclisches Amin der allgemeinen Formel VIII so kann als Säurebindemittel auch der Überschuß des Amins dienen. Auf die beschriebene Weise werden diejenigen Verbindungen der allgemeinen Formel I, in denen R = Formel III ist, bevorzugt hergestellt. Das Verfahren ist besonders in Fällen zweckmäßig, in denen das nukleophile Reagens eine geringe Reaktionsfähigkeit oder einen geringen Dampfdruck aufweist oder aus Gründen der Zugänglichkeit oder des Preises seine Anwendung im Überschuß nicht zweckmäßig ist. Ferner ist die beschriebene Verfahrensweise auch im Falle der Herstellung von Verbindungen der allgemeinen Formel I geeignet, in denen R = Formel IV, worin $R^2$ und $R^3$ unabhängig voneinander für Wasserstoff beziehungsweise Alkylreste mit 1 bis 4 Kohlenstoffatom(en), welche gegebenenfalls 2 miteinander verbunden als Alkylenrest zusammen mit dem Stickstoffatom, an welches sie gebunden sind, einen, gegebenenfalls durch 1 oder mehr weitere[s] Stickstoffatom(e) oder sonstige[s] Heteroatom(e) aufweisenden gesättigten heterocyclischen Rest darstellen, stehen. Diese Reaktion verläuft mit sehr guter Ausbeute.

Gemäß dem Verfahren a) können diejenigen Verbindungen der allgemeinen Formel I, in denen R = Formel IV, worin $R^2$ und $R^3$ beide Wasserstoff bedeuten, mit einem wasserentziehenden Mittel, zum Beispiel einem Phosphoroxyhalogenid, behandelt werden. Die Umsetzung erfolgt zweckmäßig im Überschuß des wasserentziehenden Mittels, jedoch kann als Reaktionsmedium auch ein inertes organisches aprotisches Lösungsmittel verwendet werden. Als solches kommen zum Beispiel aromatische Kohlenwasserstoffe wie Benzol, oder aliphatische Halogenkohlenwasserstoffe, wie Chloroform, in Frage. Für diese Dehydratisierung wird als Phosphoroxyhalogenid zweckmäßig Phosphoroxychlorid verwendet.

Im erfindungsgemäßen Verfahren b) wird die Nitrierung der Verbindungen der allgemeinen Formel IX direkt, mit rauchender Salpetersäure, in Gegenwart eines inerten organischen Lösungsmittels vorgenommen. Nitriert man bei 10-20 °C, vorzugsweise bei 16 °C, in Eisessig und setzt dem Reaktionsgemisch Äthanol zu, so scheidet sich das entsprechende 9-Nitro-apovincaminsäurederivat kristallin aus dem Reaktionsgemisch ab. Wird die direkte Nitrierung jedoch bei tieferen Temperaturen, zwischen —10 und 0 °C, vorgenommen und als Lösungsmittel statt Eisessig ein Halogenkohlenwasserstoff, zum Beispiel Chloroform, verwendet, so scheidet sich beim Kristallisieren des Rohproduktes aus Äthanol das entsprechende 11-Nitroapovincaminsäurederivat ab.

Die in der Estergruppe 1 oder 2 Kohlenstoffatome enthaltenden Nitroapovincaminsäureester der allgemeinen Formel I können gewünschtenfalls umgeestert werden. Die Umesterung wird zweckmäßig im Überschuß des zum Umestern verwendeten Alkohols ausgeführt, möglichst unter Ausschluß von Wasser und zweckmäßig in Gegenwart einer katalytischen Menge einer starken Base. Als Base kommen die Alkalihydride und die Alkalialkoholate, vorzugsweise tert.-Alkoholate, zum Beispiel Kalium-tert.-butylat, in Frage. Die Temperatur der Umesterung ist nicht kritisch, zweckmäßig arbeitet man bei 50-150 °C.

Die in der Estergruppe 1 oder 2 Kohlenstoffatome enthaltenden Nitroapovincaminsäureester der allgemeinen Formel I können gewünschtenfalls in an sich bekannter Weise amidiert werden. Zu diesem Zweck wird der betreffende Ester im Überschuß des Amins der allgemeinen Formel VIII vorzugsweise bis zum Siedepunkt des Amins erwärmt und dann der Überschuß des Amins durch Destillation entfernt. Diese Art der Amidbildung ist besonders dann vorteilhaft, wenn das Amin der allgemeinen Formel VIII nicht zu flüchtig ist, d. h. wenn sein Siedepunkt zwischen 70 und 150 °C liegt. Ist das Amin flüchtiger, das heißt,

daß es unterhalb von 70 °C siedet, so ist das bereits beschriebene Verfahren a) geeigneter.

In analoger Weise wird auch die Hydrazidbildung vorgenommen, d. h. die Herstellung von Verbindungen der allgemeinen Formel I, in denen R = Formel IV mit $R^2$ = Wasserstoff und $R^3$ = Aminogruppe, also R = Rest der Formel

$$-\overset{O}{\overset{\|}{C}}-\overset{H}{\overset{|}{N}}-NH_2\ ,$$

ist. Die in der Estergruppe 1 oder 2 Kohlenstoffatome enthaltenden Nitroapovincaminsäureester werden mit Hydrazin, zum Beispiel einem Überschuß an Hydrazinhydrat, umgesetzt.

Aus den erfindungsgemäßen Verbindungen der allgemeinen Formel I können gewünschtenfalls quaternäre Salze gebildet werden. Bevorzugt werden zur Quaternisierung Alkylhalogenide oder Alkylsulfate in einem geringen Überschuß eingesetzt. Die Quaternisierung wird zweckmäßig in einem inerten, organischen wasserfreien Lösungsmittel vorgenommen. Geeignet sind zum Beispiel die aliphatischen Alkohole mit 1-6 Kohlenstoffatomen, d. h. zum Beispiel wasserfreies Methanol oder Äthanol, Ketone, wie Aceton oder Methyläthylketon, ferner Acetonitril. Die Umsetzung wird zweckmäßig bei erhöhter Temperatur vorgenommen.

Die erfindungsgemäßen Verbindungen können auch zu Säureadditionssalzen umgesetzt werden. Die dafür geeigneten Säuren wurden bereits genannt. Zur Herstellung der Säureadditionssalze werden die racemisch oder optisch aktiven Verbindungen der allgemeinen Formel I in einem der oben genannten Lösungsmittel gelöst, und die Lösung wird bis zur schwach sauren Reaktion mit der entsprechenden Säure beziehungsweise mit deren mit dem gleichen Lösungsmittel bereiteter Lösung versetzt. Dann wird das ausgefallene Säureadditionssalz in geeigneter Weise, zum Beispiel durch Filtrieren, abgetrennt.

Die Erfindung betrifft sowohl die racemischen Verbindungen der allgemeinen Formel I als auch ihre optisch aktiven Isomeren. Geht man von racemischen Ausgangsstoffen aus, so wird auch als Endprodukt ein Racemat erhalten; optisch aktive Ausgangsverbindungen führen zu optisch aktiven Endprodukten. Liegt das Endprodukt als Racemat vor, so kann dieses in jeder bekannten Weise in die optisch aktiven Antipoden aufgetrennt werden. Die Trennung in optisch aktive Isomere kann in jedem beliebigen Schritt des erfindungsgemäßen Verfahrens erfolgen.

Die erfindungsgemäßen Verbindungen beziehungsweise ihre Salze können nach Beendigung des Herstellungsverfahrens gewünschtenfalls weiter gereinigt, zum Beispiel umkristallisiert werden. Das zum Umkristallisieren verwendete Lösungsmittel wird den Löslichkeits- und Kristallisationseigenschaften des jeweiligen Stoffes entsprechend gewählt.

Ferner sind erfindungsgemäß Arzneimittel, welche 1 oder mehr erfindungsgemäße Verbindung(en) und/oder 9- und/oder 11-(Nitro)-apovincamin (Formel I mit der Ergänzung, daß R für einen Kohlenwasserstoffoxycarbonylrest der allgemeinen Formel III mit $R^1$ = nicht substituierter Methylrest steht) als Wirkstoff(en), zweckmäßigerweise zusammen mit 1 oder mehr üblichen Träger- und/oder Hilfsstoff(en), enthalten, vorgesehen.

Die erfindungsgemäßen Verbindungen der allgemeinen Formel I sowie ihre physiologisch verträglichen quaternären Salze und Säureadditionssalze weisen nämlich pharmakologische, insbesondere gefäßerweiternde, spasmolytische, antihypoxische und antikonvulsive, Wirkungen auf.

Die gefäßerweiternde Wirkung wurde an mit « Chloralose-Pentobarbital »-Gemisch narkotisierten Hunden untersucht. Die Durchblutung der Gliedmaßen wurde an der Arteria femoralis gemessen, über die Durchblutung des Gehirns ergaben Messungen an der Arteria carotis interna Aufschluß. Zu den Messungen wurden elektromagnetische Strömungsmesser verwendet. Die zu untersuchenden Substanzen wurden in einer Dosis von 1 mg/kg Körpergewicht sechs Hunden appliziert, und die in den gemessenen Parametern eintretenden Veränderungen wurden in Prozent umgerechnet. Als Referenzsubstanz diente der in der Medizin als gefäßerweiternde Substanz allgemein angewendete (+)-Apovincaminsäureäthylester.

Wirkung einer Dosis von 1 mg/kg intravenös verabreichtem Wirkstoff auf den Kreislauf narkotisierter Hunde (Veränderungen in Prozent)

| Substanz | A. carotis | A. femoralis |
|---|---|---|
| Apovincaminsäureäthylester (Referenz) | +30 | +15 |
| 9-Nitroapovincaminsäureäthylester | +95 | 0 |
| 9-Nitroapovincaminsäure-(hydroxyäthyl)-ester | +45 | +10 |

Die spasmolytische Aktivität der Verbindungen wurde am isolierten Meerschweinchendarm auf klassische Weise bestimmt (Magnus, R., Pflügers Arch. 102, 123/1904/). Als Referenzsubstanzen wurden das bekannte Spasmolytikum Papaverin, ferner (+)-Apovincaminsäureäthylester verwendet.

Hemmung der durch Bariumchlorid ausgelösten Kontraktion am isolierten Meerschweinchenileum

| Substanz | $ED_{50}$ /ug/ml |
|---|---|
| Papaverin (Referenz) | 5,4 |
| Apovincaminsäureäthylester (Referenz) | 2,0 |
| 9-Nitroapovincaminsäureäthylester | 1,0 |
| 9-Nitroapovincaminsäurepropylester | 0,2 |
| 9-Nitroapovincaminsäureacetoxyäthylester | 0,4 |

Die antihypoxische Wirkung wurde an nicht narkotisierten Mäusen in normobarer Hypoxie bestimmt. Fünf männliche Mäuse wurden in einen Glaszylinder von 3 Liter Volumen gesetzt, und in den Zylinder wurde ein aus 96 % Stickstoff und 4 % Sauerstoff bestehendes Gasgemisch eingeleitet. Gemessen wurde die Zeit zwischen dem Einbringen der Tiere in den Zylinder und ihrem Verenden. Diejenigen Tiere, die die unbehandelte Kontrolle um mehr als die doppelte Zeitspanne überlebten, können als erforgreich behandelt gelten. Die zu untersuchenden Substanzen wurden je 10 Tieren in einer Dosis von 50 mg/kg intraperitoneal 30 Minuten vor Versuchsbeginn verabreicht.

Antihypoxische Wirkung der Verbindungen auf den normobarhypoxischen Zustand nicht narkotisierter Mäuse

| Substanz | Überlebensdauer+) min | % | Schutz % |
|---|---|---|---|
| Kontrolle | 6,0±1,04 | - | - |
| 9-Nitroapovincaminsäure-äthylester | 13,4 ± 2,37 | 123 | 80 |
| 9-Nitroapovincaminsäure-(acetoxyäthyl)-ester | 10,1 ± 3,51 | 68 | 30 |
| 11-Nitroapovincaminsäure-äthylester | 11,0 ± 3,35 | 83 | 50 |
| Apovincaminsäureäthylester (Referenz) | 6,4 ± 1,58 | 7 | - |
| Vincamin (Referenz) | 6,1 ± 1,30 | 2 | - |

+) durchschnittliche Lebensdauer ± Streuung (min. beziehungsweise % Verlängerung).

Die antihypoxische Wirkung wurde ferner noch an asphyxischer Anoxie (Caillard C. et al.: Life Sci. 16, 1607/1975/) und an hypobarer Hypoxie (Baumel J. et al.: Proc. Soc. Biol. N. Y. 132, 629/1969/) gemessen. Im ersteren Fall wurden die Tiere 16 Stunden lang ausgehungert, dann p.o. behandelt und eine Stunde später in gut schließende Glasgefäße von 100 ml Volumen gesetzt. Als Überlebenszeit wurde die Zeit vom Verschließen des Gefäßes bis zur letzten Atembewegung registriert. Als erfolgreich behandelt können diejenigen Tiere gelten, die eine um 30 % längere Zeit am Leben blieben als der Durchschnitt der Kontrollgruppe.

Die Wirkung auf die hypobare Hypoxie wurde an 16 Stunden lang ausgehungerten Tieren untersucht, die eine Stunde vor Versuchsbeginn p.o. mit den Verbindungen behandelt wurden. Die Tiere wurden in einen Vakuumexsikkator gesetzt, und der Druck wurde innerhalb von 20 Sekunden auf 221 mbar [170 Torr] gesenkt.

Von diesem Zeitpunkt an wurde als Überlebensdauer die Zeit bis zur letzten Atembewegung gemessen. Als erfolgreich behandelt wurden diejenigen Tiere betrachtet, deren Überlebensdauer um 100 % länger war als die durchschnittliche Überlebensdauer der Kontrolle. Aus dem prozentualen Anteil der geschützten Tiere wurden mittels Probit-Analyse die $ED_{50}$-Werte berechnet.

| Substanz | $ED_{50}$ (mg/kg, p.o.) | |
|---|---|---|
| | Asphyxische Anoxie | Hypobare Hypoxie |
| Mephenitoin (Referenz) | >80,0 | >80,0 |
| Apocincaminsäure-äthylester (Referenz) | >50,0 | >50,0 |
| 9-Nitroapovincamin-säureäthylester.HCl | 42,7 | >50,0 |

Die antikonvulsive Wirkung wurde nach folgenden Methoden untersucht.

Maximaler Elektroschock an Mäusen (Swinyard E. A. et al. : J. Pharmacol. Exp. Ther. 106, 319/1959/). Der Schock wurde an 22-24 g schweren Tieren durch eine corneale Elektrode ausgelöst (20 mA, 0,2 s, HSE-Schockgerät Typ 207). Als geschützt wurden diejenigen Tiere betrachtet, bei denen durch die Behandlung die tonische Extension der hinteren Gliedmaßen ausblieb.

Metrazolkrampf an Mäusen (Everett, G. M. and Richards, R. K. : J. Pharmacol. Exp. Ther. 81, 402/1944/). Die Tiere erhielten nach der Vorbehandlung subkutan 125 mg/kg Pentylentetrazol. Registriert wurde das Ausbleiben des persistierenden clonischen Krampfes und des tonischen Extensorkrampfes.

Strychninkrampf (Kerley, T. L. et al. : J. Pharmacol. Exp. Ther. 132, 360/1961/). Durch i.p. Applikation von 2,5 mg/kg Strychninnitrat wurde ein tonischer Extensorkrampf ausgelöst. Als geschützt wurden diejenigen überlebenden Tiere betrachtet, bei denen durch die Behandlung der Krampf ausblieb.

Die neurotoxischen Nebenwirkungen wurden auf folgende Weise untersucht.

Messung der Muskelinkoordination (Rotarod) (H. Kwibara et al. : Japan J. Pharmacol. 27, 117/1977/). Nach vorherigem Training wurden Tiere ausgewählt, die im Stande sind, 120 Sekunden lang auf der horizontalen Stange (Durchmesser 20 mm, Drehzahl 12 $min^{-1}$) oben zu bleiben. Als Muskelinkoordination wurde gewertet, wenn die Tiere innerhalb der Limitzeit herabfielen.

Muskelrelaxierende Wirkung (Traction) Männliche Mäuse eines Gewichtes von 18-22 g wurden mit den Vorderpfoten an einem horizontalen Draht von 5 mm Durchmesser aufgehängt. Verminderter Muskeltonus galt als bei denjenigen Tieren vorhanden, die ihre Hinterpfoten nicht innerhalb von 5 Sekunden nach oben neben die Vorderpfoten zogen.

| $ED_{50}$ (mg/kg, p.o.) | Mephenitoin (Referenz) | 9-Nitroapovincamin-säureäthylester.HCl +) |
|---|---|---|
| Antikonvulsive Wirkung | | |
| max. Elektroschock | 26,3 | 25,6 |
| Metrazolkrampf | 24,5 | 27,6 |
| Strychninkrampf | ∅ | 20x |
| Neurotoxische Wirkung | | |
| Rotarod | 78,6 | 180 mg/kg wirkungslos |
| Traction | 154,3 | -"- |

+) p.o. in einer Dosis von 30 mg/kg angewendet ergab sich eine Hemmwirkung von 20 %.

x prozentuale Hemmung bei einer Dosis von 30 mg/kg.

Aus diesen Experimenten ist ersichtlich, daß der Nitrosubstituent im Ring A die biologische Wirkung sehr vorteilhaft beeinflußt. Das manifestiert sich darin, daß die gefäßerweiternde Wirkung auf die Gehirngefäße wesentlich stärker ist als die der Referenzsubstanz. Im Unterschied zu der Referenzsubstanz erstreckt sich die gefäßerweiternde Wirkung spezifisch nur auf die Gehirnregion und tritt in den Blutbahnen der Extremitäten kaum oder gar nicht in Erscheinung.

Ferner zeigen die Verbindungen eine bedeutende Schutzwirkung gegen Gehirnschädigungen durch Hypoxie. Diese Wirkung kann mit einer Verbesserung des Kreislaufes verbunden sein, jedoch auch ohne diese auftreten ; es handelt sich um eine auf den Stoffwechsel des Gehirns ausgeübte Wirkung in der Richtung, daß die Gehirnzellen auch bei mangelhafter Sauerstoffversorgung fähig sind, ihre Funktionen aufrechtzuerhalten. Lebensgefährlich schwere Hypoxiezustände werden wesentlich besser toleriert.

Die Verbindungen verfügen, wie der Versuch an der glatten Darmmuskulatur zeigt, auch über eine

spasmolytische Wirkung, die die Wirkung des als Referenz verwendeten Papaverins wesentlich übertrifft. Verbindungen mit bedeutender spasmolytischer Aktivität zeigen im allgemeinen auch Kreislauf- und Antihypoxieeigenschaften, was erneut auf eine auf die Zellen oder Zellmembranen ausgeübte, insbesondere unter pathologischen Bedingungen in Erscheinung tretende, vorteilhafte pharmakologische Wirkung der erfindungsgemäßen Verbindungen hinweist.

Art und Stärke der antikonvulsiven Wirkung der Verbindungen stimmen mit der der Referenzsubstanz Mephenitoin überein, jedoch haben die erfindungsgemäßen Verbindungen auch in der sechsfachen Dosis der antikonvulsiv wirksamen Dosis keine neurotoxischen Nebenwirkungen, d. h. ihre therapeutische Breite (neurotoxischer $ED_{50}$/antikonvulsiver $ED_{50}$) ist günstiger als die der Referenzsubstanz.

Die Verbindungen der allgemeinen Formel I können mit den in der Arzneimittelherstellung üblichen, zur parenteralen oder enteralen Applikation geeigneten, nichttoxischen festen oder flüssigen Trägerstoffen und/oder Hilfsstoffen vermischt und zu Arzneimittelpräparaten formuliert werden. Als Trägerstoffe finden zum Beispiel Wasser, Gelatine, Lactose, Stärke, Pektin, Magnesiumstearat, Stearinsäure, Talk, Pflanzenöle (wie Erdnußöl und Olivenöl) Verwendung. Die Wirkstoffe werden zu den üblichen Darreichungsformen formuliert, zum Beispiel festen Präparaten (wie abgerundeten oder eckigen Tabletten, Dragees, Kapseln, z. B. Hartgelatinekapseln, Pillen und Zäpfchen) oder flüssigen Präparaten (mit Öl oder Wasser bereiteten Lösungen, Suspensionen, Emulsionen, Sirup, Weichgelatinekapseln, mit Öl oder Wasser bereiteten injizierbaren Lösungen oder Suspensionen). Die Menge des festen Trägerstoffes kann in einem weiten Bereich variieren, eine Darreichungseinheit enthält vorzugsweise etwa 25 mg-1 g Trägerstoff. Die Präparate können gegebenenfalls die üblichen Hilfsstoffe, zum Beispiel Konservierungs-, Stabilisierungs-, Netz- und Emulgiermittel, Salze zum Einstellen des osmotischen Druckes, Puffer und Geruchs- und Geschmacksstoffe, enthalten. Die Präparate können ferner weitere pharmazeutisch wertvolle, bekannte Verbindungen enthalten, ohne daß dadurch eine synergistische Wirkung einträte. Die Präparate werden vorzugsweise in Darreichungseinheiten formuliert, die der gewünschten Applikationsform entsprechen. Die Arzneimittelpräparate werden mittels der dafür üblichen Verfahren hergestellt, zum Beispiel durch Sieben, Mischen, Granulieren und Pressen der Komponenten oder durch Auflösen. Die Präparate können ferner weiteren, in der Arzneimittelindustrie üblichen Arbeitsgängen unterzogen, zum Beispiel sterilisiert werden.

Die Erfindung wird an Hand der folgenden Beispiele näher erläutert.

Beispiel 1

(+)-9-Nitroapovincamin

Ein Gemisch aus 3,6 g (0,01 Mol) (+)-9-Nitroapovincaminsäure, 50 ml Aceton, 10 ml n Natronlauge und 1 ml (0,011 Mol) Dimethylsufat wird bei Raumtemperatur 2 Stunden lang gerührt. Dann wird der pH-Wert des Gemisches mit konzentriertem Ammoniak auf 7-8 eingestellt, und das Gemisch wird in Vakuum auf etwa 2/3 seines Volumens eingedampft. Die ausgefallenen Kristalle werden abfiltriert, mit Wasser gründlich gewaschen und dann getrocknet. Man erhält 3,4 g (90 %) 9-Nitroapovincamin, das bei 160-161 °C schmilzt.

$[\alpha]_D$ = + 12,5° (c = 0,4, Pyridin). Die Spektraldaten der Verbindung stimmen mit den in der Literatur angegebenen überein.

Beispiel 2

9-Nitroapovincamin-monohydrochlorid

Ein Gemisch aus 3,6 g (0,01 Mol) wie im Beispiel 1 beschrieben erhaltener (+)-9-Nitroapovincaminsäure und 200 ml Methanol wird in Gegenwart von 2 g Molekularsieb der Porengröße 0,3 nm (3 Å) (Aldrich 20,859-2) unter Kühlen mit trockenem Salzsäuregas gesättigt. Unter fortgesetztem Einleiten von Salzsäuregas wird das Reaktionsgemisch 2 Stunden lang unter Rückfluß gekocht. Dann wird das Molekularsieb durch Filtrieren aus dem Gemisch entfernt und mit Methanol gewaschen. Die mit der Waschflüssigkeit vereinigte Lösung wird eingedampft. Man erhält als Rückstand 4,1 g (99 %) der Titelverbindung. Das rohe Produkt wird in 20 ml Aceton gelöst und durch Zusatz von 20 ml Äther kristallisiert. Die Kristalle werden mit einem im Volumverhältnis 1 : 1 aus Äther und Aceton bereiteten Lösungsmittelgemisch gewaschen und dann getrocknet. Ausbeute: 3,4 g (82 %), Schmelzpunkt: 185-188 °C.

Beispiel 3

(+)-11-Nitroapovincamin

Man arbeitet auf die im Beispiel 1 beschriebene Weise, geht jedoch von 3,6 g (0,01 Mol) (+)-11-Nitroapovincaminsäure aus und erhält 3,2 g (85 %) der Titelverbindung, die bei 137-139 °C schmilzt.

$[\alpha]_D$ = + 31,25° (c = 0,4, Pyridin).

Die Spektraldaten der Verbindung stimmen mit den in der Literatur angegebenen überein.

Beispiel 4

11-Nitroapovincamin

Man arbeitet auf die im Beispiel 2 beschriebene Weise, geht jedoch von 3,6 g (0,01 Mol) (+)-11-Nitroapovincaminsaüre aus. Die erhaltenen 3,9 g (95 %) 11-Nitroapovincaminmonohydrochlorid werden in einem Gemisch aus 50 ml Wasser und 50 ml Äthylacetat mit konzentriertem wäßrigem Ammoniak versetzt. Die wäßrige Phase wird abgetrennt und zweimal mit je 30 ml Äthylacetat extrahiert. Die vereinigten organischen Phasen werden dreimal mit je 20 ml Wasser gewaschen, über geglühtem Natriumsulfat getrocknet und dann eingedampft. Der Rückstand kristallisiert beim Verreiben mit Äther. Man erhält 3,2 g (85 %) der Titelverbindung, die bei 137-139 °C schmilzt.

Beispiel 5

9-Nitroapovincaminsäure-äthylester-monohydrochlorid

Ein Gemisch aus 3,6 g (0,01 Mol) (+)-9-Nitroapovincaminsäure und 200 ml wasserfreiem Äthanol wird in Gegenwart von 2 g eines Molekularsiebes der Porenweite 0,3 (3 Å) nm (Aldrich 20,859-2) unter Kühlen mit trockenem Salzsäuregas gesättigt. Unter fortgesetztem Einleiten von Salzsäuregas wird das Reaktionsgemisch 2 Stunden lang am Rückfluß gekocht. Das Molekularsieb wird abfiltriert und mit Äthanol gewaschen. Das mit der Waschflüssigkeit vereinigte Filtrat wird eingedampft und der Rückstand (4,3 g ; 99 %) mit 10 ml Aceton verrieben. Die Kristalle werden abfiltriert, mit 5 ml Aceton gewaschen und dann getrocknet. Man erhält 3,7 g (88 %) der Titelverbindung, die bei 220-224 °C schmilzt.

Elementaranalyse für $C_{22}H_{26}N_3O_4Cl$ (M = 431,99)

| | | | | |
|---|---|---|---|---|
| berechnet, %: | C 61,16 | H 6,06 | N 9,72, | Cl 8,22 |
| gefunden, %: | C 61,14 | H 6,60 | N 9,57 | Cl 8,11 |

Beispiel 6

(+)-9-Nitroapovincaminsäure-äthylester

Die Lösung von 9,0 g (0,023 Mol) wie im Beispiel 1 beschrieben erhaltenem (+)-9-Nitroapovincamin in 250 ml wasserfreiem Äthanol wird in Gegenwart von 0,3 g Kalium-tert.-butylat 2 Stunden lang unter Rückfluß gekocht. Das Reaktionsgemisch wird zur Trockne eingedampft und der Rückstand in einem Gemisch aus 350 ml Dichlormethan und 100 ml Wasser gelöst. Die organische Phase wird abgetrennt, zweimal mit je 100 ml Wasser gewaschen, über wasserfreiem Natriumsulfat getrocknet und dann eingedampft. Der ölige Eindampfrückstand erstarrt bei Verreiben mit 50 ml Äther zu gelben Kristallen. Diese werden abfiltriert, mit Äther gewaschen und dann getrocknet. Man erhält 8,3 g (91 %) der Titelverbindung, die bei 125-126 °C schmilzt.

$[\alpha]_D = + 241,9°$ (c = 1, Chloroform)

$^1$H-NMR-Spektrum ($CDCl_3$) δ : Et 1,02(t)3, 1,93(q)2 ; Et0 1,39(t)3, 4,40(q)2 ; H-3 4,11(s)1 ; H-15 6,28(s)1 ; H-10 7,85(dd)1 (J = 8 und 1 Hz) ; H-11 7,10(t)1 (J = 8 Hz) ; H-12 7,41(dd)1 (J = 8 und 1 Hz) Gerüstprotonen 1,3-3,4(m)10.

Beispiel 7

(+)-11-Nitroapovincaminsäure-äthylester

Ein Gemisch aus 7,3 g (0,02 Mol) (+)-11-Nitroapovincaminsäure, 100 ml Aceton, 20 ml n Natronlauge und 3 ml (0,023 Mol) Diäthylsulfat wird bei Raumtemperatur 3 Stunden lang gerührt. Dann wird das Gemisch mit konzentriertem Ammonial auf pH 8 alkalisch gestellt und im Vakuum eingedampft. Die ausgefallenen Kristalle werden abfiltriert, mit Wasser gewaschen und dann getrocknet. Man erhält 6,8 g (86 %) der Titelverbindung, die bei 166-167 °C schmilzt.

$[\alpha]_D = + 110,98°$ (c = 0,4, Chloroform)

$^1$H-NMR-Spektrum ($CDCl_3$) δ : Et 1,02(t)3, 1,93q(2), Et0 1,41t(3), 4,46q(2), H-3 4,11s(1), H-15 6,30 s(1), H-9 7,41d(1) (J = 8,8 Hz), H-10 7,96dd(1) (J = 8,8 und 2 Hz), H-12 8,2d(1) (J = 2 Hz), Gerüstprotonen 1,66-3,5 m(10).

Beispiel 8

11-Nitroapovincaminsäure-äthylester-monohydrochlorid

Die Lösung von 4 g (0,011 Mol) (+)-Apovincaminsäure-äthylester in 20 ml Chloroform wird auf — 5 °C gekühlt und bei dieser Temperatur mit einem Gemisch aus 4 ml rauchender Salpetersäure (spez. Gew. 1,52) und 16 ml Eisessig versetzt. Das Gemisch wird bei Temperaturen zwischen — 5 °C und — 2 °C 30 Minuten lang gerührt, dann auf 200 g zerstoßenes Eis gegossen und mit 20 ml Chloroform versetzt. Unter Rühren wird das Gemisch mit konzentriertem Ammoniak auf pH 8 alkalisch gestellt. Die wäßrige Phase wird abgetrennt, zweimal mit je 10 ml Chloroform extrahiert, die vereinigten organischen Phasen werden dreimal mit je 20 ml Wasser gewaschen, über geglühtem Natriumsulfat getrocknet und nach dem Filtrieren eingedampft. Der Eindampfrückstand (4,3 g) wird in 20 ml Äthanol heiß gelöst und die Lösung zwei Tage lang im Kühlschrank stehengelassen. Die Kristalle werden abgetrennt und mit 5 ml Äthanol

gewaschen. Man erhält 1,3 g (30 %) 11-Nitroapovincaminsäure-äthylester, der bei 182-186 °C schmilzt. Aus dem Produkt wird in Aceton mit salzsaurem Äthanol das Monohydrochlorid bereitet.

Elementaranalyse für $C_{22}H_{26}N_3O_4Cl$ (M = 431,99)

| | | | | |
|---|---|---|---|---|
| berechnet, %: | C 61,16 | H 6,06 | N 9,72, | Cl 8,22 |
| gefunden, %: | C 60,98 | H 6,24 | N 9,74 | Cl 8,13 |

Beispiel 9

(+)-11-Nitroapovincaminsäure-äthylester

7,3 g (0,02 Mol) (+)-11-Nitroapovincaminsäure und 0,12 g Kaliumhydroxyd werden in 20 ml wasserfreiem Äthanol gelöst. Aus der Lösung wird der Alkohol verdampft, das zurückgebliebene gelbe kristalline Kaliumsalz wird in 25 ml Acetonitril suspendiert und die Suspension mit 0,18 ml Äthyljodid versetzt. Das Gemisch wird unter Rühren 3 Stunden lang unter Rückfluß gekocht und dann das Lösungsmittel im Vakuum abdestilliert. Der Rückstand wird in einem Gemisch aus 50 ml Wasser und 50 ml Äthylacetat gelöst und die Lösung auf pH 8 alkalisch gestellt. Nach gründlichem Schütteln werden die Phasen voneinander getrennt. Die wäßrige Phase wird zweimal mit je 20 ml Äthylacetat gewaschen. Die vereinigten organischen Phasen werden dreimal mit je 10 ml Wasser gewaschen, über geglühtem Natriumsulfat getrocknet und nach dem Filtrieren zur Trockne eingedampft. Der Rückstand wird mit 10 ml Äther verrieben, die gelben Kristalle werden abfiltriert und mit Äther gewaschen. Man erhält 6,8 g (86 %) der Titelverbindung, die bei 166-167 °C schmilzt.

$[\alpha]_D = + 110,98°$ (c = 0,4, Chloroform)

Beispiel 10

11-Nitroapovincaminsäurechlorid-monohydrochlorid

3,67 g (0,01 Mol) (+)-11-Nitroapovincaminsäure werden in 10 ml Thionylchlorid 90 Minuten lang gekocht. Die erhaltene Lösung wird im Vakuum zur Trockne eingedampft. Der Rückstand wird mit 10 ml wasserfreiem Benzol versetzt und erneut zur Trockne eingedampft. Bei Verreiben mit 10 ml Äther bilden sich Kristalle, die abfiltriert und mit Äther gewaschen werden. Man erhält 4,1 g (97 %) der Titelverbindung, die bei 224 — 117 °C schmilzt.

Beispiel 11

11-Nitroapovincaminsäure-(3'-dimethylamino-propylester)-dihydrochlorid

1,6 g (0,0038 Mol) des gemäß Beispiel 10 hergestellten Säurechlorids werden zusammen mit 1,3 ml (0,011 Mol) 3-Dimethylaminopropanol in 10 ml Chloroform bei Raumtemperatur gerührt. Nach einer halben Stunder werden 10 ml Chloroform und 5 ml Wasser zugegeben, und die Phasen werden voneinander getrennt. Die organische Phase wird dreimal mit je 5 ml Wasser gewaschen, über geglühtem Natriumsulfat getrocknet und das Lösungsmittel nach dem Filtrieren abdestilliert. Man erhält 1,6 g (93,5 %) der Basenform der Titelverbindung in Form einer öligen Substanz.

$^1$H-NMR ($CDCl_3$) δ: Et 1,03t(3), 1,93q(2), $N(CH_3)_2$ 2,22 s(6), H-3 4,13 s(1), $OCH_2$ 4,46 t(2), H-15 6,32 s(t), H-9 7,32 d(1) (J = 9 Hz), H-10 7,97 dd(1) (J = 9 und 2 Hz), H-12 8,22 d(1), (J = 2 Hz), Gerüstprotonen +N—$CH_2CH_2$ 1,10-3,44 m(14).

Die 1,6 g Base werden in 20 ml Aceton gelöst, die Lösung wird mit salzsaurem Äthanol auf pH 4 angesäuert, das ausgefallene Salz wird abfiltriert und mit Aceton gewaschen. Man erhält 1,27 g (64 %) der Titelverbindung, die bei 171-176 °C unter Zersetzung schmilzt.

Elementaranalyse für $C_{25}H_{32}N_4O_4H_2Cl_2$ (M = 525,35)

| | | | | |
|---|---|---|---|---|
| berechnet, %: | C 57,15 | H 10,65 | N 6,47, | Cl 13,49 |
| gefunden, %: | C 57,10 | H 10,62 | N 6,60 | Cl 13,56 |

Beispiel 12

11-Nitroapovincaminsäure-benzylester und sein Monohydrochlorid

Auf die im Beispiel 10 beschriebene Weise wird aus (+)-11-Nitroapovincaminsäure das Säurechlorid hergestellt. 1,6 g (0,0038 Mol) 11-Nitroapovincaminsäurechlorid-monohydrochlorid werden in 10 ml Benzylalkohol gelöst. Die Lösung wird bei 100 °C drei Stunden lang gerührt und nach dem Abkühlen mit 10 ml Wasser verdünnt. Die Lösung wird mit konzentriertem Ammoniak auf pH 8 alkalisch gestellt und dann dreimal mit je 20 ml Äthylacetat extrahiert. Die vereinigten Extrakte werden zweimal mit je 10 ml gesättigter wäßriger Kochsalzlösung und dann mit 10 ml Wasser gewaschen. Das Lösungsmittel wird im Vakuum entfernt. Man erhält 1,66 g (95,9 %) der Basenform der Titelverbindung.

$^1$H-NMR ($CDCl_3$) δ: Et 1,00 t(3), 0,92 q(2), H-3 4,10 s(1), $OCH_2$ 5,34 d(1) und 5,50 d(1) ($J_{gem}$ = 12 Hz), H-15 6,35 s(1), H-10 7,95 dd(1) (J = 9 Hz und 2 Hz), H-12 8,22 d(1) (J = 2 Hz), sonstiges Ar-H 7,20-7,50 m(6), Gerüstprotonen 1,10-3,40 m(10).

Die Base wird in 100 ml Äther gelöst und die Lösung mit salzsaurem Äthanol auf pH 4 angesäuert. Die ausgefallene Substanz wird abfiltriert, mit Äther gewaschen und aus 20 ml Äthanol umkristallisiert. Man erhält 0,99 g (53 %) der Titelverbindung, die bei 146-150 °C schmilzt.

Elementaranalyse für $C_{27}H_{27}N_3O_4.HCl$ (M = 493,92)

| | | | | |
|---|---|---|---|---|
| berechnet, %: | C 65,65 | H 8,50 | N 5,66 | Cl 7,17 |
| gefunden, %: | C 65,48 | H 8,54 | N 5,82 | Cl 7,26 |

Beispiel 13

11-Nitroapovincaminsäure-(3'-pyridylmethylester)

Auf die im Beispiel 10 beschriebene Weise wird 11-Nitroapovincaminsäurechlorid-hydrochlorid hergestellt. 1,6 g (0,0038 Mol) des Hydrochlorids werden zusammen mit 1,5 ml (0,015 Mol) 3-Pyridylmethanol in 20 ml Chloroform bei Raumtemperatur 3 Stunden lang gerührt. Dann wird das Gemisch mit 10 ml Wasser versetzt und mit gesättigter wäßriger Natriumcarbonatlösung auf pH 8 alkalisch gestellt. Die organische Phase wird abgetrennt, dreimal mit je 10 ml Wasser gewaschen, über geglühtem Natriumsulfat getrocknet und im Vakuum zur Trockne eingedampft. Der ölige Rückstand wird in 10 ml Methanol heiß gelöst, die Lösung wird geklärt und dann gekühlt. Die ausgefallenen Kristalle werden abfiltriert, mit kaltem Methanol gewaschen und dann getrocknet. Man erhält 1,42 g (82 %) der Titelverbindung, die bei 136-138 °C schmilzt.

$^1$H-NMR ($CDCl_3$) δ: Et 1,10 t(3), 1,92 q(2), H-3 4,15 s(1), $CH_2$-0 5,38 d(1) und 5,48 d(1) ($J_{gem}$ = 12 Hz), H-15 6,36 s(1), H-9 7,44 d(1) (J = 9 Hz), H-10 7,98 dd(1) (J = 9 und 2 Hz), H-12 8,16 d(1) (J = 2 Hz), Ar-H (Pyridin) 8,68 dd(1), 8,58 dd(1), 7,82 t(1), 7,33 ddd(1), Gerüstprotonen 1,20-3,40 m(10).

Elementaranalyse für $C_{26}H_{26}N_4O_4$ (m = 458,46)

| | | | |
|---|---|---|---|
| berechnet, %: | C 68,11 | N 12,21 | H 5,67 |
| gefunden, %: | C 68,25 | N 12,08 | H 5,58 |

Beispiel 14

11-Nitroapovincaminsäure-(2'-hydroxyäthylester)

Ein Gemisch aus 1,85 g (0,0049 Mol) wie im Beispiel 1 beschrieben erhaltenem (+)-11-Nitroapovincamin, 0,1 g Kalium-tert.-butylat und 60 ml Äthylenglykol wird bei 120 °C 90 Minuten lang gerührt und nach dem Abkühlen mit 30 ml Wasser versetzt. Die ausgefallenen Kristalle werden abfiltriert, viermal mit je 25 ml Wasser gewaschen und dann getrocknet. Man erhält 1,9 g (95 %) der Titelverbindung, die bei 227-229 °C schmilzt.

Beispiel 15

9-Nitroapovincaminsäure-(2'-hydroxyäthylester)

Ein Gemisch aus 1,85 g (0,0049 Mol) wie im Beispiel 1 beschrieben erhaltenem (+)-9-Nitroapovincamin, 0,1 g Kalium-tert.-butylat und 60 ml Äthylenglykol wird bei 120 °C 90 Minuten lang gerührt und dann im Vakuum auf 3/4 seines Volumens eingedampft. Der Rückstand wird in 50 ml Äthylacetat gelöst, die Lösung dreimal mit je 20 ml Wasser gewaschen, über geglühtem Natriumsulfat getrocknet und nach dem Filtrieren eingedampft. Der Rückstand wird mit Äther verrieben. Die Kristalle werden abfiltriert und mit Äther gewaschen. Man erhält 1,47 g (74 %) der Titelverbindung, die bei 152-154 °C schmilzt.

$^1$H-NMR ($CDCl_3$) δ: Et 1,00t(3), 1,90q(2), H—3 3,92s(1), $CH_2OCO$ 4,45m(2), HO—$CH_2$— 3,90m(2), H—15 6,32s(1), Ar—H 7,18—8,00m(3).

Beispiel 16

9-Nitroapovincaminsäure-(2'-methylpropylester) und sein Monohydrochlorid

Ein Gemisch aus 1 g (0,0026 Mol) wie im Beispiel 1 beschrieben erhaltenem (+)-9-Nitroapovincamin, 30 ml wasserfreiem Isobutanol und 0,01 g Kalium-tert.-butylat wird eine halbe Stunde lang unter Rückfluß gekocht und dann im Vakuum zur Trockne eingedampft. Der Rückstand wird in 50 ml Äthylacetat gelöst, die Lösung dreimal mit je 20 ml Wasser gewaschen, filtriert und dann eingedampft. Der Rückstand (1,09 g, 99 %) ist die Basenform der Titelverbindung.

$^1$H-NMR ($CDCl_3$) δ: Ar-H 7,06-8,1 m(3), H-15 6,34 s(1), H-3 4,12 s(1), -$CH_2$-O 4,20 d(2).

Die Base wird in 10 ml Aceton gelöst und die Lösung mit salzsaurem Äthanol auf pH 4 angesäuert. Die ausfallenden gelben Kristalle werden abfiltriert, mit kaltem Aceton gewaschen und dann getrocknet. Man erhält 0,73 g (62 %) der Titelverbindung, die bei 222-224 °C schmilzt.

Elementaranalyse für $C_{24}H_{29}N_3O_4 \cdot HCl$ (M = 459,89)

| | | | | |
|---|---|---|---|---|
| berechnet, %: | C 62,67 | H 6,30 | N 9,13 | Cl 7,70 |
| gefunden, %: | C 62,76 | H 6,80 | N 9,10 | Cl 7,87. |

Beispiel 17

9-Nitroapovincaminsäure-octylester-monohydrochlorid

Die Lösung von 1 g (0,0026 Mol) wie im Beispiel 1 beschrieben erhaltenem (+)-9-Nitroapovincamin in 10 ml Caprylalkohol wird in Gegenwart von 0,01 g Kaliumtert.-butylat 20 Stunden lang auf 140 °C gehalten. Dann wird das Gemisch im Vakuum zur Trockne eingedampft, der Rückstand in 50 ml Äthylacetat gelöst und die Lösung dreimal mit je 20 ml n Salzsäure extrahiert. Die organische Phase wird zur Trockne eingedampft, der Rückstand in 10 ml Aceton gelöst und die Lösung mit salzsaurem Äthanol auf pH 3 angesäuert. Nach Zusatz von 10 ml Diisopropyläther läßt man das Gemisch über Nacht im Kühlschrank stehen. Die Kristalle werden abfiltriert, mit Äther gewaschen und dann getrocknet. Man erhält 0,71 g (53 %) der Titelverbindung, die bei 168-172 °C schmilzt.

Elementaranalyse für $C_{28}H_{37}N_3O_4 \cdot HCl$ (M = 516,04)

| | | | | |
|---|---|---|---|---|
| berechnet, %: | C 65,11 | H 7,36 | N 8,13 | Cl 6,86 |
| gefunden, %: | C 65,45 | H 7,82 | N 8,12 | Cl 6,90. |

Beispiel 18

9-Nitroapovincaminsäure-propylester-monohydrochlorid

Ein Gemisch aus 1,5 g (0,004 Mol) wie im Beispiel 1 beschrieben erhaltenem (+)-9-Nitroapovincamin, 150 ml wasserfreiem Propylalkohol und 0,1 g Kalium-tert.-butylat wird 10 Stunden lang gekocht und dann zur Trockne eingedampft. Der Rückstand wird in 60 ml Äthylacetat gelöst und die Lösung dreimal mit je 20 ml Wasser gewaschen. Nach dem Trocknen über wasserfreiem Natriumsulfat und Filtrieren wird die organische Phase im Vakuum zur Trockne eingedampft. Der Rückstand (1,5 g, 92 %) wird in einem Gemisch aus 2 ml Aceton und 20 ml Äther gelöst und die Lösung mit salzsaurem Äthanol auf pH 3 angesäuert. Die Kristalle werden abfiltriert, mit Äther gewaschen und dann getrocknet. Man erhält 1,34 g (76 %) der Titelverbindung, die bei 199-204 °C schmilzt.

Elementaranalyse für $C_{23}H_{27}N_3O_4 \cdot HCl$ (M = 446,92)

| | | | | |
|---|---|---|---|---|
| berechnet, %: | C 61,89 | H 6,27 | N 9,41 | Cl 7,95 |
| gefunden, %: | C 62,00 | H 6,46 | N 9,40 | Cl 7,88. |

Beispiel 19

9-Nitroapovincaminsäureamid und sein Monohydrochlorid

Ein Gemisch aus 1,84 g (0,005 Mol) (+)-9-Nitroapovincaminsäure und 5 ml Thionylchlorid wird unter Rühren eine Stunde lang unter Rückfluß gekocht. Die Lösung wird zur Trockne eingedampft und der Rückstand mit 20 ml Benzol versetzt. Die ausgefallenen Kristalle werden abfiltriert, dreimal mit je 10 ml Benzol gewaschen und dann getrocknet. Auf diese Weise erhält man 1,6 g (74 %) 9-Nitroapovincaminsäurechlorid-hydrochlorid, das bei 238-240 °C schmilzt. Das Hydrochlorid wird in kleinen Portionen in 100 ml Äther eingetragen, der vorher mit Ammoniakgas gesättigt wurde. Während der Zugabe wird gerührt und mit Eis gekühlt. Das Gemisch wird zwei Stunden lang bei 0 °C und zwei weitere Stunden lang bei Raumtemperatur gerührt. Der pH-Wert wird durch Zugabe von ätherischem Ammoniak auf 8-9 gehalten. Die ausgefallenen Kristalle werden abfiltriert und mit Äther, danach dreimal mit je 20 ml Wasser gewaschen. Man erhält 1,37 g (75 %) der Basenform der Titelverbindung, die bei 270-274 °C schmilzt.

Die Base wird in 5 ml Äthanol suspendiert und der pH-Wert der Suspension mit salzsaurem Äthanol auf 3 eingestellt. Nach Zusatz von 2 ml Äther fallen Kristalle aus, die abfiltriert, mit Äther gewaschen und dann getrocknet werden. Man erhält 1,62 g (81 %) der Titelverbindung, die bei 256-260 °C schmilzt.

Elementaranalyse für $C_{20}H_{22}N_4O_3 \cdot HCl$ (M = 402,87)

| | | | | |
|---|---|---|---|---|
| berechnet, %: | C 59,62 | H 5,75 | N 13,9 | Cl 8,79 |
| gefunden, %: | C 59,32 | H 6,00 | N 14,0 | Cl 8,65. |

Beispiel 20

9-Nitroapovincaminsäurenitril und sein Monohydrochlorid

Ein Gemisch aus 3,66 g (0,01 Mol) wie im Beispiel 19 beschrieben erhaltenem 9-Nitroapovincaminsäureamid und 10 ml Phosphoroxychlorid wird unter Rückfluß gekocht und danach zur Trockne eingedampft. Der Rückstand wird in 100 ml Dichloräthan gelöst und die Lösung mit n Natronlauge auf pH 8 alkalisch gestellt. Die organische Phase wird abgetrennt, mit 30 ml Wasser gewaschen, über geglühtem Natriumsulfat getrocknet und nach dem Filtrieren zur Trockne eingedampft. Der Rückstand wird in 15 ml Methanol warm gelöst und die Lösung dann gekühlt. Die ausgefallenen Kristalle werden abfiltriert, mit kaltem Methanol gewaschen und dann getrocknet. Man erhält 2,7 g (77 %) der Titelverbindung, die bei 139-142 °C schmilzt.

$^1$H-NMR ($CDCl_3$) δ : Et 1,04 t(3), 1,91 q(2), H-3 4,24 s(1), H-15 6,15 s(1), H-10 7,95 dd(1), H-11 7,29 t(1),

H-12 8,35 dd(1), Gerüstprotonen 0,90-3,40 m(10).

0,35 g (0,001 Mol) 9-Nitroapovincaminsäurenitril werden in 5 ml Aceton gelöst, und die Lösung wird mit salzsaurem Äthanol auf pH 3 angesäuert. Nach Zugabe von 2 ml Äther fallen Kristalle aus, die abfiltriert, mit Äther gewaschen und getrocknet werden. Man erhält 0,28 g (73 %) des Hydrochlorids, das bei 219-222 °C schmilzt.

Elementaranalyse für $C_{20}H_{20}N_4O_2 \cdot HCl$ (M = 383,86)

| | | | | |
|---|---|---|---|---|
| berechnet, %: | C 62,42 | H 5,24 | N 14,56 | Cl 9,21 |
| gefunden, %: | C 62,56 | H 5,87 | N 14,63 | Cl 9,80. |

Beispiel 21

11-Nitroapovincaminsäure-diäthylamid

Ein Gemisch aus 0,42 g (0,001 Mol) wie im Beispiel 10 beschrieben erhaltenem 11-Nitroapovincaminsäurechlorid-hydrochlorid, 10 ml Dichloräthan und 2 ml Diäthylamin wird bei Raumtemperatur eine Stunde lang gerührt und dann dreimal mit je 20 ml Wasser extrahiert. Die organische Phase wird über geglühtem Natriumsulfat getrocknet und nach dem Filtrieren zur Trockne eingedampft. Der Rückstand wird aus einem Gemisch von 3 ml Methanol und 5 ml Äther kristallisiert. Man erhält 0,29 g (69 %) der Titelverbindung, die bei 140-141 °C schmilzt.

Elementaranalyse für $C_{24}H_{30}N_4O_3$ (M = 422,51)

| | | | |
|---|---|---|---|
| berechnet, %: | C 68,22 | H 7,16 | N 13,26 |
| gefunden, %: | C 68,40 | H 7,88 | N 13,30. |

Beispiel 22

9-Nitroapovincaminsäure-(4'-methylpiperidid) und sein Monohydrochlorid

Zu der Suspension von 2,11 g (0,005 Mol) wie im Beispiel 19 beschrieben erhaltenem 9-Nitroapovincaminsäurechlorid-hydrochlorid in 25 ml wasserfreiem Benzol wird unter Rühren bei Raumtemperatur die Lösung von 2,4 ml (0,04 Mol) 4-Methylpiperidin in 10 ml Benzol zugegeben. Das Gemisch wird unter Rückfluß eine Stunde lang gekocht und dann im Vakuum zur Trockne eingedampft. Der Rückstand wird in einem Gemisch aus 50 ml Äthylacetat und 50 ml Wasser gelöst. Die organische Phase wird abgetrennt, zweimal mit je 30 ml Wasser gewaschen, über geglühtem Natriumsulfat getrocknet und nach dem Filtrieren zur Trockne eingedampft. Der Rückstand wird aus 20 ml Äther kristallisiert. Man erhält 1,86 g (83 %) der Basenform der Titelverbindung, die bei 198-200 °C schmilzt.

Die Base wird in 50 ml Äther gelöst und der pH-Wert der Lösung unter Rühren mit salzsaurem Äthanol auf 3 eingestellt. Das Gemisch wird bei Raumtemperatur zwei Stunden lang gerührt. Dann wird der Niederschlag abfiltriert, mit Äther gewaschen und dann getrocknet. Man erhält 1,69 g (70 %) des Hydrochlorids, das bei 216-220 °C schmilzt.

Elementaranalyse für $C_{26}H_{32}N_4O_3 \cdot HCl$ (M = 485,02)

| | | | | |
|---|---|---|---|---|
| berechnet, %: | C 64,38 | H 6,85 | N 11,55 | Cl 7,31 |
| gefunden, %: | C 64,47 | H 7,01 | N 11,65 | Cl 7,28 |

Beispiel 23

11-Nitroapovincaminsäure-phenylester

Ein Gemisch aus 1,0 g (0,0027 Mol) 11-Nitroapovincaminsäure und 10 ml Thionylchlorid wird eine Stunde lang unter Rückfluß gekocht und dann der Überschuß des Thionylchlorids im Vakuum abdestilliert. Der Rückstand wird mit 20 ml wasserfreiem Benzol versetzt und im Vakuum erneut zur Trockne eingedampft. Dieser Arbeitsgang wird noch einmal wiederholt. Der Rückstand wird in 10 ml wasserfreiem Aceton gelöst und die Lösung bei 0 °C tropfenweise zu einer Lösung gegeben, die aus 0,28 g (0,003 Mol) Phenol, 10 ml Aceton und 6 ml n Natronlauge bereitet wurde. Das Gemisch wird bei 0 °C zwei Stunden lang gerührt und dann im Vakuum zur Trockne eingedampft. Der Rückstand wird in 50 ml Äthylacetat gelöst und die Lösung dreimal mit je 20 ml Wasser extrahiert. Die organische Phase wird über wasserfreiem Natriumsulfat getrocknet und nach dem Filtrieren zur Trockne eingedampft. Man erhält 0,63 g (52 %) der Titelverbindung, die bei 96-98 °C schmilzt.

Beispiel 24

11-Nitroapovincaminsäure-hydrazid

Ein Gemisch aus 0,48 g (0,0013 Mol) 11-Nitroapovincaminsäure und 5 ml Thionylchlorid wird eine Stunde lang unter Rückfluß gekocht und dann der Überschuß des Thionylchlorids im Vakuum abdestilliert. Zu dem Rückstand werden 10 ml wasserfreies Benzol gegeben und dann im Vakuum

abgedampft. Dieser Arbeitsgang wird noch einmal wiederholt. Der Eindampfrückstand wird in 5 ml wasserfreiem Aceton gelöst. Die Lösung wird unter Rühren bei 0 °C in die Lösung von 7 ml Hydrazinhydrat in 10 ml Aceton eingetropft. Das Gemisch wird bei 0 °C noch eine Stunde lang gerührt und dann im Vakuum zur Trockne eingedampft. Der Rückstand wird in 50 ml Äthylacetat gelöst und die Lösung dreimal mit je 30 ml Wasser extrahiert. Die organische Phase wird über geglühtem Natriumsulfat getrocknet und nach dem Filtrieren zur Trockne eingedampft. Der Rückstand kristallisiert bei Verreiben mit Hexan. Die Kristalle werden abfiltriert, mit Hexan gewaschen und dann getrocknet. Man erhält 0,34 g (68 %) der Titelverbindung, die bei 143-146 °C schmilzt.

## Beispiel 25

9-Nitroapovincaminsäure-propargylester-monohydrochlorid

2,0 g (0,0048 Mol) des nach der Verfahrensweise des Beispieles 10 hergestellten Säurechlorids werden bei Raumtemperatur unter Rühren zu der Lösung von 0,23 g metallischem Matrium in 5 ml Propargylalkohol gegeben. Das Gemisch wird bei Raumtemperatur eine Stunde lang geruhrt und dann eingedampft. Der Rückstand wird in 50 ml Äthylacetat aufgenommen und dreimal mit je 20 ml Wasser gewaschen. Die organische Phase wird über geglühtem Natriumsulfat getrocknet und dann eingedampft. 1,6 g (82 %) der Basenform der Titelverbindung werden in Form einer öligen Substanz erhalten.

Die Base wird in 10 ml Aceton gelöst und die Lösung nach Zusatz von 10 ml Äther mit salzsaurem Äthanol auf pH 4 angesäuert. Die ausgefallenen Kristalle werden abfiltriert, mit einem im Volumverhältnis von 1 : 1 bereiteten Gemisch von Äther und Aceton gewaschen und dann getrocknet. Man erhält 1,6 g (75 %) der Titelverbindung, die bei 205-208 °C schmilzt.

## Beispiel 26

9-Nitroapovincaminsäure-2′-trifluoräthylester-monohydrochlorid

Ein Gemisch aus 2,0 g (0,0048 Mol) des nach der Verfahrensweise des Beispieles 10 hergestellten Säurechlorids, 10 ml Trifluoräthanol und 2 ml Triäthylamin wird bei Raumtemperatur eine Stunde lang gerührt. Das Gemisch wird eingedampft, der Rückstand in 50 ml Äthylacetat gelöst und die Lösung dreimal mit je 20 ml Wasser gewaschen. Die organische Phase wird über wasserfreiem Natriumsulfat getrocknet und dann eingedampft. Als Rückstand erhält man 1,55 g der Basenform der Titelverbindung. Die Base wird in einem Gemisch aus 10 ml Aceton und 50 ml Äther gelöst und der pH-Wert der Lösung mit salzsaurem Äthanol auf 4 eingestellt. Die ausgefallenen Kristalle werden abfiltriert und mit Äther gewaschen. Man erhält 1,58 g (68 %) der Titelverbindung, die bei 200-205 °C schmilzt.

## Beispiel 27

(+)-9-Nitroapovincaminsäure-allylester-monohydrochlorid

2 g (0,0052 Mol) wie im Beispiel 1 beschrieben erhaltenes (+)-9-Nitroapovincamin, 25 ml wasserfreier Allylalkohol und 0,01 g Kalium-tert.-butylat werden 8 Stunden lang unter Rückfluß gekocht. Das Gemisch wird im Vakuum zur Trockne eingedampft, der Rückstand in 50 ml Äthylacetat gelöst und die Lösung dreimal mit je 20 ml Wasser gewaschen. Die organische Phase wird über wasserfreiem Natriumsulfat getrocknet und dann im Vakuum zur Trockne eingedampft. Man erhält 1,9 g (90 %) der Basenform der Titelverbindung in Form einer öligen Substanz. Die Base wird in 50 ml Äther gelöst und der pH-Wert der Lösung mit salzsaurem Äthanol auf 4 eingestellt. Die ausgefallenen gelben Kristalle werden abfiltriert, mit Äther gewaschen und dann getrocknet. Man erhält 1,6 g (69 %) der Titelverbindung, die bei 193-197 °C schmilzt.

$[\alpha]_D^{20} = + 264,97°$ (c = 0,2, Äthanol).

## Beispiel 28

(+)-9-Nitroapovincaminsäure-propylester-4-methojodid

Zu der Lösung von 1,5 g wie im Beispiel 18 beschrieben erhaltenem (+)-9-Nitroapovincaminsäure-propylester in 10 ml Aceton werden 1,2 ml Methyljodid gegeben. Das Gemisch wird bei Raumtemperatur eine Stunde lang gerührt und dann über Nacht im Kühlschrank stehengelassen. Die gelben Kristalle werden abfiltriert, mit Aceton gewaschen und dann getrocknet. Man erhält 1,32 g (68 %) der Titelverbindung, die bei 190-192 °C schmilzt.

$[\alpha]_D^{20} = + 116,64°$ (c = 2,0, Chloroform).

## Beispiel 29

9-Nitroapovincaminsäure-cyclopentylester-monohydrochlorid

Zu einem Gemisch von 15 ml wasserfreiem Benzol und 5 ml Cyclopentanol werden 0,23 g metallisches Natrium gegeben, und das Gemisch wird unter Rückfluß so lange gekocht, bis das Natrium in Lösung gegangen ist. Zu der auf Raumtemperatur abgekühlten Lösung werden unter Rühren 2,0 g

(0,0048 Mol) nach der Verfahrensweise des Beispieles 10 hergestelltes 9-Nitroapovincaminsäurechlorid-hydrochlorid gegeben. Das Reaktionsgemisch wird eine Stunde lang unter Rückfluß gekocht, abgekühlt und mit 50 ml Äthylacetat versetzt. Dann wird das Gemisch dreimal mit je 20 ml Wasser gewaschen, die organische Phase wird abgetrennt, über wasser freiem Natriumsulfat getrocknet und im Vakuum zur Trockne eingedampft. Als Rückstand erhält man 1,3 g der Basenform der Titelverbindung. Die Base wird in 50 ml Äther gelöst und der pH-Wert der Lösung mit salzsaurem Äthanol auf 4 eingestellt. Die gelben Kristalle werden abfiltriert, mit Äther gewaschen und getrocknet. Man erhält 1,06 g (49 %) der Titelverbindung, die bei 210-215 °C unter Zersetzung schmilzt.

**Patentansprüche** (für die Vertragsstaaten : BE, CH, DE, FR, GB, IT, LI, NL, SE)

1. 9-beziehungsweise 11-(Nitro)-apovincaminsäurederivate der allgemeinen Formel

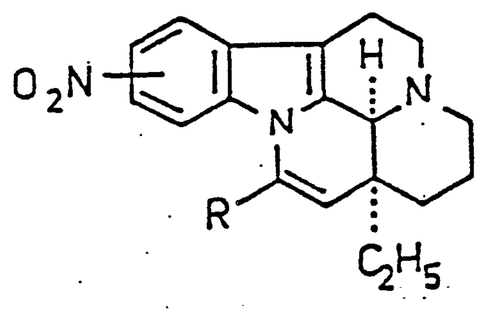

I

worin R für einen Halogencarbonylrest der allgemeinen Formel

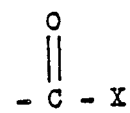

II

in welchletzterer X ein Halogenatom bedeutet, oder einen Kohlenwasserstoffoxycarbonylrest der allgemeinen Formel

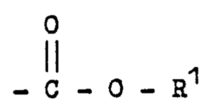

III

in welchletzterer $R^1$ einen aliphatischen Kohlenwasserstoffrest mit 1 bis 10 Kohlenstoffatom(en), cycloaliphatischen Kohlenwasserstoffrest mit 3 bis 8 Kohlenstoffatomen oder aromatischen Kohlenwasserstoffrest mit 6 bis 14 Kohlenstoffatomen bedeutet,
wobei ein derartiger Rest bzw. derartige Reste durch [ein] Halogenatom(e), [eine] primäre, sekundäre und/oder tertiäre Aminogruppe(n), [eine] Hydroxygruppe(n) [einen] Arylrest(e) mit 6 bis 14 Kohlenstoffatomen [ein] Alkyl-, Alkoxy- und/oder Alkylthiorest(e) mit [je] 1 bis 8 Kohlenstoffatom(en), [ein] Trifluormethylrest(e), [eine] Carboxylgruppe(n), [einen] Alkoxycarbonylrest(e) mit 1 bis 8 Kohlenstoffamtom(en) im Alkylteil, [eine] Thio-, Sulfinyl-Sulfonyl-, Nitro-, Keto-, Aldehyd- und/oder Cyangruppe(n) und/oder [einen] 1 oder mehr gleiche oder verschiedene Heteroatom(e) aufweisende(n) Heteroarylrest(e) sowie gegebenenfalls zusätzlich zu diesen Gliedern mit 4 zur Bildung eines Benzolrings ankondensierten Gliedern, der bzw. die gegebenenfalls durch 1 oder mehr der aufgeführten Substituenten substituiert sein kann bzw. können, substituiert sein kann [können] oder einen Aminocarbonylrest der allgemeinen Formel

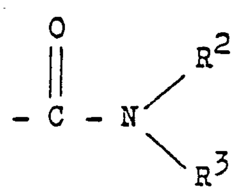

IV

in welchletzterer
$R^2$ und $R^3$ unabhängig voneinander Wasserstoff beziehungsweise Alkylreste mit 1 bis 8 Kohlenstoffatom(en), welche gegebenenfalls 2 miteinander verbunden als Alkylenrest zusammen mit dem Stickstoffatom, an welches sie gebunden sind, einen, Pyrrolidino-, Piperidino-, Imidazolidino-, Piperazino-, oder

Morpholinorest darstellen, bedeuten, oder

$R^2$ Wasserstoff bedeutet und

$R^3$ eine Aminogruppe darstellt,

oder eine Cyanogruppe steht und die Nitrogruppe in der 9- oder 11-Stellung ist, mit der weiteren Maßgabe, daß

R von einem Kohlenwasserstoffoxycarbonylrest der allgemeinen Formel III, bei welchem $R^1$ einen nicht substituierten Methylrest bedeutet, verschieden ist,

sowie ihre optisch aktiven Isomere und Salze.

2. 9-beziehungsweise 11-(Nitro)-apovincaminsäurederivate nach Anspruch 1, dadurch gekennzeichnet, daß das Halogenatom, für welches X steht, ein Chlor- oder Bromatom ist.

3. 9-beziehungsweise 11-(Nitro)-apovincaminsäurederivate nach Anspruch 1, dadurch gekennzeichnet, daß der aliphatische Kohlenwasserstoffrest, für welchen $R^1$ stehen kann, ein Alkyl-, Alkenyl- oder Alkinylrest ist.

4. 9-beziehungsweise 11-(Nitro)-apovincaminsäurederivate nach Anspruch 1, dadurch gekennzeichnet, daß der cycloaliphatische Kohlenwasserstoffrest, für welchen $R^1$ stehen kann, ein Cycloalkylrest ist, beziehungsweise der aromatische Kohlenwasserstoffrest, für welchen $R^1$ stehen kann, monocyclisch, nämlich der Phenylrest, oder polycyclisch nicht kondensiert oder polycyclisch kondensiert ist.

5. 9-beziehungsweise 11-(Nitro)-apovincaminsäurederivate nach Anspruch 1, 3 oder 4, dadurch gekennzeichnet, daß der beziehungsweise die Substituent(en), durch welche[n] der aliphatische, cycloaliphatische beziehungsweise aromatische Kohlenwasserstoffrest, für den $R^1$ steht, substituiert sein kann, [eine] Dialkylaminogruppe(n) mit 1 bis 10 Kohlenstoffatom(en) in jedem Alkylteil, [ein] Phenyl-, Diphenyl- oder Naphthylrest(e), [ein] Alkyl-, Alkoxy- und/oder Alkylthiorest(e) mit [je] 1 bis 4 Kohlenstoffatom(en), [ein] Alkoxycarbonylrest(e) mit 1 bis 4 Kohlenstoffatom(en) im Alkylteil, [ein] Pyrrolyl-, Furyl-, Thienyl-, Pyridyl-, Pyranyl-, Pyrazolyl-, Imidazolyl-, Pyrimidinyl-, Indolyl-, und/oder Chinolylrest(e) ist beziehungsweise sind.

6. 9-beziehungsweise 11-(Nitro)-apovincaminsäurederivate nach Anspruch 1, dadurch gekennzeichnet, daß der beziehungsweise die Alkylrest(e), für welche [n] $R^2$ und/oder $R^3$ stehen kann beziehungsweise können, [ein] solche[r] mit 1 bis 4 Kohlenstoffatom(en) ist beziehungsweise sind.

7. Die 9-beziehungsweise 11-(Nitro)-apovincaminsäurederivate nach Anspruch 1, 9-(Nitro)-apovincaminsäure-äthylester, 11-(Nitro)-apovincaminsäure-äthylester, 9-(Nitro)-apovincaminsäurechlorid, 11-(Nitro)-apovincaminsäurechlorid, 11-[Nitro]-apovincaminsäure-{[(dimethylamino)-n-propyl]-ester}, insbesondere 11-{[Nitro]-apovincaminsäure-[3'-(dimethylamino)-n-propyl]-ester}, 9-[Nitro]-apovincaminsäure-{[(dimethylamino)-n-propyl]-ester}, insbesondere 9-[Nitro]-apovincaminsäure-{[3'-(dimethylamino)-n-propyl]-ester}, 11-(Nitro)-apovincaminsäure-benzylester, 9-(Nitro)-apovincaminsäure-benzylester, 11-(Nitro)-apovincaminsäure-pyridylmethylester, insbesondere 11-(Nitro)-apovincaminsäure-{[3'-(pyridyl)-methyl]-ester}, 9-(Nitro)-apovincaminsäure-pyridylmethylester, insbesondere 9-(Nitro)-apovincaminsäure-{[3'-(pyridyl)-methyl]-ester}, 9-(Nitro)-apovincaminsäure-(hydroxyäthylester), insbesondere 9-(Nitro)-apovincaminsäure-{[2'-(hydroxy)-äthyl]-ester}, 11-(Nitro)-apovincaminsäure-(hydroxyäthylester), insbesondere 11-(Nitro)-apovincaminsäure-{[2'-(hydroxy)-äthyl]-ester}, 9-(Nitro)-apovincaminsäure-isobutylester, 11-(Nitro)-apovincaminsäure-isobutylester, 9-(Nitro)-apovincaminsäure-n-octylester, 11-(Nitro)-apovincaminsäure-n-octylester, 9-(Nitro)-apovincaminsäure-n-propylester, 11-(Nitro)-apovincaminsäure-n-propylester, 9-(Nitro)-apovincaminsäurenitril, 11-(Nitro)-apovincaminsäurenitril, 9-(Nitro)-apovincaminsäureamid, 11-(Nitro)-apovincaminsäureamid, 9-(Nitro)-apovincaminsäure-diäthylamid, 11-(Nitro)-apovincaminsäure-diäthylamid, 9-(Nitro)-apovincaminsäure-[4'-(methyl)-piperidid], 11-(Nitro)-apovincaminsäure-[4'-(methyl)-piperidid], 11-(Nitro)-apovincaminsäure-phenylester, 9-(Nitro)-apovincaminsäure-phenylester, 11-(Nitro)-apovincaminsäure-hydrazid, 9-(Nitro)-apovincaminsäure-hydrazid, 9-(Nitro)-apovincaminsäure-propargylester, 11-(Nitro)-apovincaminsäure-propargylester, 9-[Nitro]-apovincaminsäure-{[2'-tri-(fluor)-äthyl]-ester}, 11-[Nitro]-apovincaminsäure-{[2'-tri-(fluor)-äthyl]-ester}, 9-(Nitro)-apovincaminsäure-allylester, 11-(Nitro)-apovincaminsäure-allylester, 9-(Nitro)-apovincaminsäure-cyclopentylester und 11-(Nitro)-apovincaminsäure-cyclopentylester sowie ihre optisch aktiven Isomere und Hydrochloride.

8. Verfahren zur Herstellung der 9- beziehungsweise 11-(Nitro)-apovincaminsäurederivate nach Anspruch 1 bis 7 beziehungsweise von 9- beziehungsweise 11-(Nitro)-apovincamin, dadurch gekennzeichnet, daß man

a) racemische oder optisch aktive 9- beziehungsweise 11-(Nitro)-apovincaminsäuren beziehungsweise Derivate derselben der allgemeinen Formel

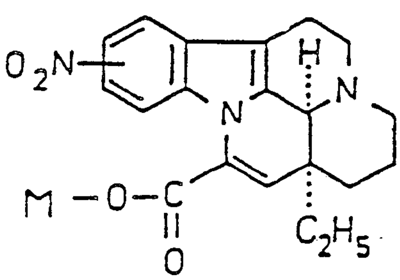

worin M für Wasserstoff, ein Metallatom oder eine Ammoniumgruppe steht, oder, falls M von einem Metallatom verschieden ist, auch deren Säureadditionssalze beziehungsweise quaternäre Salze mit Hydroxyverbindungen beziehungsweise Estern der allgemeinen Formel

$$R^1 — Y \qquad VI,$$

worin

$R^1$ die in den Ansprüchen 1 oder 3 bis 5 angegebenen Bedeutungen hat oder einen nicht substituierten Methylrest darstellt und

Y für eine Hydroxygruppe oder einen Säurerest steht,

gegebenenfalls in Gegenwart von Basen, oder mit Halogenierungsmitteln umsetzt und gegebenenfalls die erhaltenen 9- beziehungsweise 11-(Nitro)-apovincaminsäurederivate der allgemeinen Formel I, bei welchen R für einen Halogencarbonylrest der allgemeinen Formel II steht, mit nukleophilen Reagenzien der allgemeinen Formel

$$H—O—R^1 \qquad VII$$

beziehungsweise

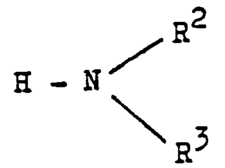

VIII

worin $R^1$, $R^2$ und $R^3$ die im Anspruch 1 oder 3-bis 6 angegebenen Bedeutungen haben oder $R^1$ für einen nicht substituierten Methylrest steht, umsetzt und gegebenenfalls die erhaltenen 9- beziehungsweise 11-(Nitro)-apovincaminsäurederivate der allgemeinen Formel I, bei welchen R für einen Aminocarbonylrest der allgemeinen Formel IV, bei welchem $R^2$ und $R^3$ Wasserstoffatome bedeuten, steht, mit wasserentziehenden Mitteln dehydratisiert oder

b) racemische oder optisch aktive Apovincaminsäurederivate der allgemeinen Formel

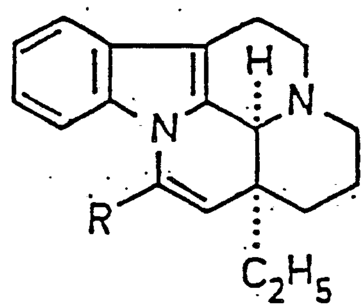

IX

worin R die in den Ansprüchen 1 bis 6 angegebenen Bedeutungen hat, oder deren Säureadditionssalze nitriert, sowie gegebenenfalls die erhaltenen 9- beziehungsweise 11-(Nitro)-apovincaminsäurederivate der allgemeinen Formel I, bei welchen R für einen Kohlenwasserstoffoxycarbonylrest der allgemeinen Formel III, bei welchem $R^1$ einen Alkylrest mit 1 oder 2 Kohlenstoffatom(en) bedeutet, steht, beziehungsweise ihre Säureadditionssalze umestert oder amidiert und/oder gegebenenfalls die erhaltenen 9-beziehungsweise 11-(Nitro)-apovincaminsäurederivate der allgemeinen Formel I in Säureadditionssalze oder quaternäre Salze überführt und/oder gegebenenfalls die erhaltenen Salze der 9- beziehungsweise 11-(Nitro)-apovincaminsäurederivate der allgemeinen Formel I in die freien 9- beziehungsweise 11-(Nitro)-apovincaminsäurederivate der allgemeinen Formel I oder in andere Säureadditionssalze beziehungsweise quaternäre Salze überführt und/oder gegebenenfalls eine Spaltung der erhaltenen racemischen 9-beziehungsweise 11-(Nitro)-apovincaminsäurederivate der allgemeinen Formel I beziehungsweise von deren Säureadditionssalzen beziehungsweise quaternären Salzen beziehungsweise eine Racemisierung der entsprechenden optisch aktiven Verbindungen in Stereoisomere vornimmt.

9. Arzneimittel, gekennzeichnet durch einen Gehalt an 1 oder mehr Verbindung(en) nach Anspruch 1 bis 7 und/oder 9- und/oder 11-(Nitro)-apovincamin als Wirkstoff(en), zweckmäßigerweise zusammen mit 1 oder mehr üblichen Träger- und/oder Hilfsstoff(en).

**Patentansprüche** (für den Vertragsstaat AT)

1. Verfahren zur Herstellung von 9- bzw. 11-(Nitro)-apovincaminsäurederivaten der allgemeinen Formel

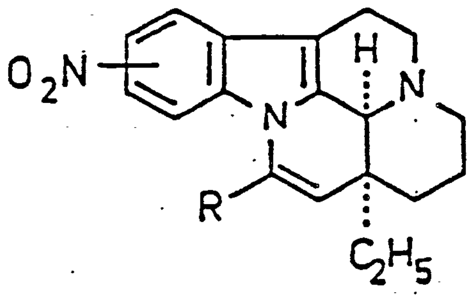

I

worin R für einen Halogencarbonylrest der allgemeinen Formel

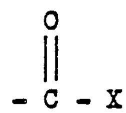

II

in welchletzterer X ein Halogenatom bedeutet, oder einen Kohlenwasserstoffoxycarbonylrest der allgemeinen Formel

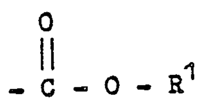

III

in welchletzterer $R^1$ einen aliphatischen Kohlenwasserstoffrest mit 1 bis 10 Kohlenstoffatom(en), cycloaliphatischen Kohlenwasserstoffrest mit 3 bis 8 Kohlenstoffatomen oder aromatischen Kohlenwasserstoffrest mit 6 bis 14 Kohlenstoffatomen bedeutet,
wobei ein derartiger Rest bzw. derartige Reste durch [ein] Halogenatom(e), [eine] primäre, sekundäre und/oder tertiäre Aminogruppe(n), [eine] Hydroxygruppe(n) [einen] Arylrest(e) mit 6 bis 14 Kohlenstoffatomen, [ein] Alkyl-, Alkoxy- und/oder Alkylthiorest(e) mit [je] 1 bis 8 Kohlenstoffatom(en), [ein] Trifluormethylrest(e), [eine] Carboxylgruppe(n), [einen] Alkoxycarbonylrest(e) mit 1 bis 8 Kohlenstoffamtom(en) im Alkylteil, [eine] Thio-, Sulfinyl-, Sulfonyl-, Nitro-, Keto-, Aldehyd-, und/oder Cyangruppe(n) und/oder [einen] 1 oder mehr gleiche oder verschiedene Heteroatom(e) aufweisende(n) Heteroarylrest(e) sowie gegebenenfalls zusätzlich zu diesen Gliedern mit 4 zur Bildung eines Benzolrings ankondensierten Gliedern, der bzw. die gegebenenfalls durch 1 oder mehr der aufgeführten Substituenten substituiert sein kann bzw. können, substituiert sein kann [können] oder einen Aminocarbonylrest der allgemeinen Formel

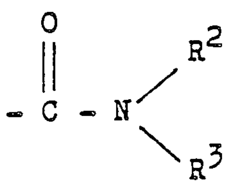

IV

in welchletzterer

$R^2$ und $R^3$ unabhängig voneinander Wasserstoff beziehungsweise Alkylreste mit 1 bis 8 Kohlenstoffatom(en), welche gegebenenfalls 2 miteinander verbunden als Alkylenrest zusammen mit dem Stickstoffatom, an welches sie gebunden sind, einen, Pyrrolidino-, Piperidino-, Imidazolidino-, Piperazino-, oder Morpholinorest darstellen, bedeuten, oder

$R^2$ Wasserstoff bedeutet und

$R^3$ eine Aminogruppe darstellt,

oder eine Cyanogruppe steht und die Nitrogruppe in der 9- oder 11- Stellung ist, mit der weiteren Maßgabe, daß

R von einem Kohlenwasserstoffoxycarbonylrest der allgemeinen Formel III, bei welchem $R^1$ einen nicht substituierten Methylrest bedeutet, verschieden ist,

sowie ihren optisch aktiven Isomeren und Salzen, dadurch gekennzeichnet, daß man

a) racemische oder optisch aktive 9- beziehungsweise 11-(Nitro)-apovincaminsäuren beziehungsweise Derivate derselben der allgemeinen Formel

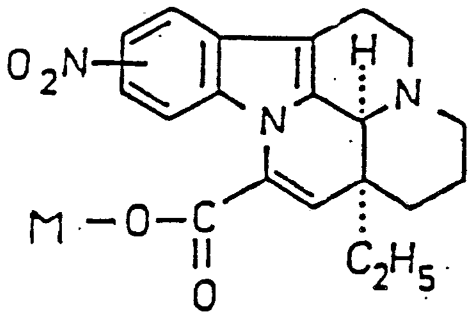

V

worin M für Wasserstoff, ein Metallatom oder eine Ammoniumgruppe steht, oder, falls M von einem Metallatom verschieden ist, auch deren Säureadditionssalze beziehungsweise quaternäre Salze mit Hydroxyverbindungen beziehungsweise Estern der allgemeinen Formel

$$R^1—Y \qquad VI,$$

worin

$R^1$ die oben angegebenen Bedeutungen hat oder einen nicht substituierten Methylrest darstellt und

Y für eine Hydroxygruppe oder einen Säurerest steht,

gegebenenfalls in Gegenwart von Basen, oder mit Halogenierungsmitteln umsetzt und gegebenenfalls die erhaltenen 9- beziehungsweise 11-(Nitro)-apovincaminsäurederivate der allgemeinen Formel I, bei welchen R für einen Halogencarbonylrest der allgemeinen Formel II steht, mit nukleophilen Reagenzien der allgemeinen Formel

$$H—O—R^1 \qquad VII$$

beziehungsweise

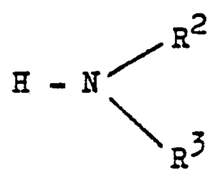

VIII

worin $R^1$, $R^2$ und $R^3$ die oben angegebenen Bedeutungen haben oder $R^1$ für einen nicht substituierten Methylrest steht, umsetzt und gegebenenfalls die erhaltenen 9- beziehungsweise 11-(Nitro)-apovincaminsäurederivate der allgemeinen Formel I, bei welchen R für einen Aminocarbonylrest der allgemeinen Formel IV, bei welchem $R^2$ und $R^3$ Wasserstoffatome bedeuten, steht, mit wasserentziehenden Mitteln dehydratisiert oder

b) racemische oder optisch aktive Apovincaminsäurederivate der allgemeinen Formel

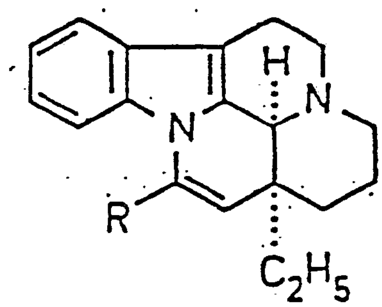

IX

worin R die oben angegebenen Bedeutungen hat, oder deren Säureadditionssalze nitriert, sowie gegebenenfalls die erhaltenen 9- beziehungsweise 11-(Nitro)-apovincaminsäurederivate der allgemeinen Formel I, bei welchen R für einen Kohlenwasserstoffoxycarbonylrest der allgemeinen Formel III, bei welchem $R^1$ einen Alkylrest mit 1 oder 2 Kohlenstoffatom(en) bedeutet, steht, beziehungsweise ihre Säureadditionssalze umestert oder amidiert und/oder gegebenenfalls die erhaltenen 9- beziehungsweise 11-(Nitro)-apovincaminsäurederivate der allgemeinen Formel I in Säureadditionssalze oder quaternäre Salze überführt und/oder gegebenenfalls die erhaltenen Salze der 9- beziehungsweise 11-(Nitro)-apovincaminsäurederivate der allgemeinen Formel I in die freien 9- beziehungsweise 11-(Nitro)-apovincaminsäurederivate der allgemeinen Formel I oder in andere Säureadditionssalze beziehungsweise quaternäre Salze überführt und/oder gegebenenfalls eine Spaltung der erhaltenen racemischen 9- beziehungsweise 11-(Nitro)-apovincaminsäurederivate der allgemeinen Formel I beziehungsweise von deren Säureadditionssalzen beziehungsweise quaternären Salzen beziehungsweise eine Racemisierung der entsprechenden optisch aktiven Verbindungen in Stereoisomere vornimmt.

2. Verfahren nach Anspruch 1, dadurch gekennzeichnet, daß Derivate der Formel I hergestellt werden, in denen das Halogenatom, für welches X steht, ein Chlor- oder Bromatom ist.

3. Verfahren nach Anspruch 1, dadurch gekennzeichnet, daß Derivate der Formel I hergestellt werden, in denen der aliphatische Kohlenwasserstoffrest, für welchen $R^1$ stehen kann, ein Alkyl-, Alkenyl- oder Alkinylrest ist.

4. Verfahren nach Anspruch 1, dadurch gekennzeichnet, daß Derivate der Formel I hergestellt werden, in denen der cycloaliphatische Kohlenwasserstoffrest, für welchen $R^1$ stehen kann, ein Cycloalkylrest ist, beziehungsweise der aromatische Kohlenwasserstoffrest, für welchen $R^1$ stehen kann, monocyclisch, nämlich der Phenylrest, oder polycyclisch nicht kondensiert oder polycyclisch kondensiert ist.

5. Verfahren nach Anspruch 1, 3 oder 4, dadurch gekennzeichnet, daß Derivate der Formel I hergestellt werden, in denen der beziehungsweise die Substituent(en), durch welche[n] der aliphatische, cycloaliphatische beziehungsweise aromatische Kohlenwasserstoffrest, für den $R^1$ steht, substituiert sein kann, [eine] Dialkylaminogruppe(n) mit 1 bis 10 Kohlenstoffatom(en) in jedem Alkylteil, [ein] Phenyl-, Diphenyl- oder Naphthylrest(e), [ein] Alkyl-, Alkoxy- und/oder Alkylthiorest(e) mit [je] 1 bis 4 Kohlenstoffatom(en), [ein] Alkoxycarbonylrest(e) mit 1 bis 4 Kohlenstoffatom(en) im Alkylteil, [ein] Pyrrolyl-, Furyl-, Thienyl-, Pyridyl-, Pyranyl-, Pyrazolyl-, Imidazolyl-, Pyrimidinyl-, Indolyl- und/oder Chinolylrest(e) ist beziehungsweise sind.

6. Verfahren nach Anspruch 1, dadurch gekennzeichnet, daß Derivate der Formel I hergestellt werden, in denen der bzw. die Alkylrest(e), für welche(n) $R^2$ und/oder $R^3$ stehen kann bzw. können, (ein) solche(r) mit 1 bis 4 Kohlenstoffatom(en) ist beziehungsweise sind.

7. Verfahren nach Anspruch 1, dadurch gekennzeichnet, daß 9-(Nitro)-apovincaminsäure-äthylester, 11-(Nitro)-apovincaminsäure-äthylester, 9-(Nitro)-apovincaminsäurechlorid, 11-(Nitro)-apovincaminsäurechlorid, 11-[Nitro]-apovincaminsäure-{[(dimethylamino)-n-propyl]-ester}, insbesondere 11-[Nitro]-apovincaminsäure-{[3'-(dimethylamino)-n-propyl]-ester}, 9-[Nitro]-apovincaminsäure-{[(dimethylamino)-n-propyl]-ester}, insbesondere 9-[Nitro]-apovincaminsäure-{[3'-(dimethylamino)-n-propyl]-ester}, 11-(Nitro)-apovincaminsäure-benzylester, 9-(Nitro)-apovincaminsäure-benzylester, 11-(Nitro)-apovincaminsäure-pyridylmethylester, insbesondere 11-(Nitro)-apovincaminsäure-{[3'-(pyridyl)-methyl]-ester}, 9-(Nitro)-apovincaminsäure-pyridylmethylester, insbesondere 9-(Nitro)-apovincaminsäure-{[3'-(pyridyl)-methyl]-ester}, 9-(Nitro)-apovincaminsäure-(hydroxyäthylester), insbesondere 9-(Nitro)-apovincaminsäure-{[2'-(hydroxy)-äthyl]-ester}, 11-(Nitro)-apovincaminsäure-(hydroxyäthylester), insbesondere 11-(Nitro)-apovincaminsäure-{[2'-(hydroxy)-äthyl]-ester}, 9-(Nitro)-apovincaminsäure-isobutylester, 11-(Nitro)-apovincaminsäure-isobutylester, 9-(Nitro)-apovincaminsäure-n-octylester, 11-(Nitro)-apovincaminsäure-n-octylester, 9-(Nitro)-apovincaminsäure-n-propylester, 11-(Nitro)-apovincaminsäure-n-propylester, 9-(Nitro)-apovincaminsäurenitril, 11-(Nitro)-apovincaminsäurenitril, 9-(Nitro)-apovincaminsäureamid, 11-(Nitro)-apovincaminsäureamid, 9-(Nitro)-apovincaminsäure-diäthylamid, 11-(Nitro)-apovincaminsäure-diäthylamid, 9-[Nitro]-apovincaminsäure-[4'-(methyl)-piperidid], 11-[Nitro]-apovincaminsäure-[4'-(methyl)-piperidid], 11-(Nitro)-apovincaminsäure-phenylester, 9-(Nitro)-apovincaminsäure-phenylester, 11-(Nitro)-apovincaminsäure-hydrazid, 9-(Nitro)-apovincaminsäure-hydrazid, 9-(Nitro)-apovincaminsäure-propargylester, 11-(Nitro)-apovincaminsäure-propargylester, 9-[Nitro]-apovincaminsäure-{[2'-tri-(fluor)-äthyl]-ester}, 11-[Nitro]-apovincaminsäure-{[2'-tri-(fluor)-äthyl]-ester}, 9-(Nitro)-apovincaminsäure-allylester, 11-(Nitro)-apovincaminsäure-allylester, 9-(Nitro)-apovincaminsäure-cyclopentylester und 11-(Nitro)-apovincaminsäure-cyclopentylester sowie ihre optisch aktiven Isomere und Hydrochloride hergestellt werden.

**Claims** (for the Contracting States : BE, CH, DE, FR, GB, IT, LI, NL, SE)

1. 9- and/or 11-(nitro)-apovincamid acid derivatives of the general formula

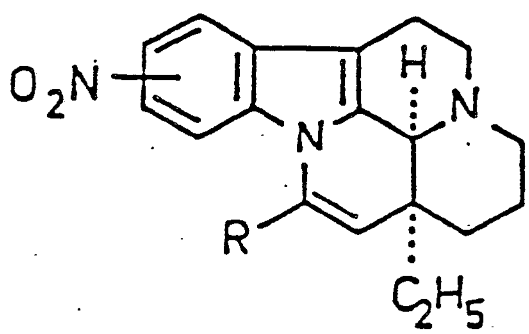

wherein R stand for a halogencarbonyl residue of the general formula

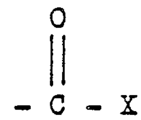

II

in which X is a halogen atom or for a hydrocarbonoxycarbonyl residue of the general formula

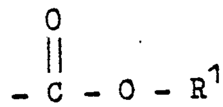

III

in which $R^1$ is an aliphatic hydrocarbon residue having 1 to 10 carbon atom(s), or a cycloaliphatic hydrocarbon residue having 3 to 8 carbon atoms or an aromatic hydrocarbon residue having 6 to 14 carbon atoms
wherein such residue or residues may be substituted by (one) halogen atom(s), (one) primary, secondary and/or tertiary amino group(s), (one) hydroxy group(s), (one) aryl residue(s) having 6 to 14 carbon atoms, (one) alkyl, alkoxy and/or alkylthio residue(s) (each) having 1 to 8 carbon atom(s), (one) trifluoromethyl residue(s), (one) carboxyl group(s), (one) alkoxycarbonyl residue(s) having 1 to 8 carbon atom(s) in the alkyl moiety, (one) thio, sulfinyl, sulfonyl, nitro, keto, aldehyde and/or cyan group(s) and/or (one) heteroalkyl residue(s) having one or more identical or different heteroatom(s) and optionally in addition to said members with 4 fused-on members for forming a benzene ring, which may optionally be substituted by one or more of the substituents listed or for an aminocarbonyl residue of the general formula

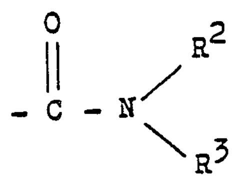

IV

wherein

$R^2$ and $R^3$ independently of each other are hydrogen and/or alkyl residues having 1 to 8 carbon atom(s), two of which may optionally be interconnected to form an alkylene residue and together with the nitrogen atom to which they are bonded constitute a pyrrolidino, piperidino, imidazolidino, piperazino or morpholino residue, or

$R^1$ is hydrogen and

$R^3$ is an amino group,

or for a cyano group and the nitro group is in porision 9 or 11, provided that

R is different from a hydrocarbonoxycarbonyl residue of the general formula III in which $R^1$ is an unsubstituted methyl residue,

and their optically active isomers and salts.

2. 9- and/or 11-(nitro)-apovincamic acid derivatives according to claim 1, characterized in that the halogen atom represented by X is a chlorine or bromine atom.

3. 9- and/or 11-(nitro)-apovincamic acid derivatives according to claim 1, characterized in that the aliphatic hydrocarbon residue which may be represented by $R^1$ is an alkyl, alkenyl or alkinyl residue.

4. 9- and/or 11-(nitro)-apovincamic acid derivatives according to claim 1, characterized in that the cycloaliphatic hydrocarbon residue which may be represented by $R^1$ is a cycloalkyl residue or the aromatic hydrocarbon residue which may be represented by $R^1$ is monocyclic, namely the phenyl residue, or is polycyclic and uncondensed or polycyclic and condensed.

5. 9- or 11-(nitro)-apovincamic acid derivatives according to claim 1, 3 or 4, characterized in that the substituent or substituents with which the aliphatic, cycloaliphatic or aromatic hydrocarbon residue which may be represented by $R^1$ may be substituted is or are (one) dialkylamino group(s) having 1 to 10 carbon atom(s) in each alkyl moiety, (one) phenyl, diphenyl or naphthyl residue(s), (one) alkyl, alkoxy and/or alkylthio residue(s) (each) having 1 to 4 carbon atom(s), (one) alkoxycarbonyl residue(s) having 1 to 4 carbon atom(s) in the alkyl moiety, (one) pyrrolyl, furyl, thienyl, pyridyl, pyranyl, pyrazolyl, imidazolyl, pyrimidinyl, indolyl and/or quinolyl residue(s).

6. 9- and/or 11-(nitro)-apovincamic acid derivatives according to claim 1, characterized in that the alkyl residue(s) which may be represented by $R^2$ and/or $R^3$ has or have 1 to 4 carbon atom(s).

7. The 9- and/or 11-(nitro)-apovincamic acid derivatives according to claim 1 9-(nitro)-apovincamic acid ethyl esters, 11-(nitro)-apovincamic acid ethyl esters, 9-(nitro)-apovincamic acid chloride, 11-(nitro)-apovincamic acid chloride, 11-[nitro]-apovincamic acid {[(dimethylamino)-n-propyl] esters}, particularly

11-[nitro]-apovincamic acid [3'-(dimethylamino)-n-propyl esters], 9-[nitro]-apovincamic acid {[(dimethylamino)-n-propyl] esters}, particularly 9-[nitro]-apovincamic acid {[3'-(dimethylamino)-n-propyl ester}, 11-(nitro)-apovincamic acid benzyl esters, 9-(nitro)-apovincamic acid benzyl esters, 11-(nitro)-apovincamic acid pyridylmethyl esters, particularly 11-(nitro)-apovincamic acid {[3'-(pyridyl)-methyl] ester}, 9-(nitro)-apovincamic acid pyridylmethyl esters, particularly 9-(nitro)-apovincamic acid {[3'-(pyridyl)-methyl] ester}, 9-(nitro)-apovincamic acid (hydroxyethyl esters), particularly 9-(nitro)-apovincamic acid {[2'-hydroxy)-ethyl ester}, 11-(nitro)-apovincamic acid (hydroxyethyl esters), particularly 11-(nitro)-apovincamic acid, {[2'-(hydroxy)-ethyl] ester}, 9-(nitro)-apovincamic acid isobutyl esters, 11-(nitro)-apovincamic acid isobutyl esters, 9-(nitro)-apovincamic acid n-octyl esters, 11-(nitro)-apovincamic acid n-octyl esters, 9-(nitro)-apovincamic acid n-propyl esters, 11-(nitro)-apovincamic acid -propyl esters, 9-(nitro)-apovincamic acid nitrile, 11-(nitro)-apovincamic acid nitrile, 9-(nitro)-apovincamic acid amide, 9-(nitro)-apovincamic acid amide, 11-(nitro)-apovincamic acid amide, 9-(nitro)-apovincamic acid diethylamide, 11-(nitro)-apovincamic acid diethylamide, 9-[nitro]-apovincamic acid [4'-(methyl)-piperidide], 11-[nitro]-apovincamic acid [4'-(methyl)-piperidide], 11-(nitro)-apovincamic acid phenyl esters, 9-(nitro)-apovincamic acid phenyl esters, 11-(nitro)-apovincamic acid hydrazide, 9-(nitro)-apovincamic acid hydrazide, 9-(nitro)-apovincamic acid propargyl ester, 11-(nitro)-apovincamic acid propargyl ester, 9-[nitro]-apovincamic acid {[2'-tri-(fluoro)-ethyl] esters}, 11-[nitro]-apovincamic acid {[2'-tri-(fluoro)-ethyl] esters}, 9-(nitro)-apovincamic acid allyl esters, 11-(nitro)-apovincamic acid allyl esters, 9-(nitro)-apovincamic acid cyclopentyl esters and 11-(nitro)-apovincamic acid cyclopentyl esters and their optically active isomers and hydrochlorides.

8. A process of producing the 9- and/or 11-(nitro)-apovincamic acid derivatives according to claims 1 to 7 or of 9- and/or 11-(nitro)-apovincamine, characterized in that

a) racemic or optically active 9- and/or 11-(nitro)-apovincamic acids and/or derivatives thereof, which have the general formula

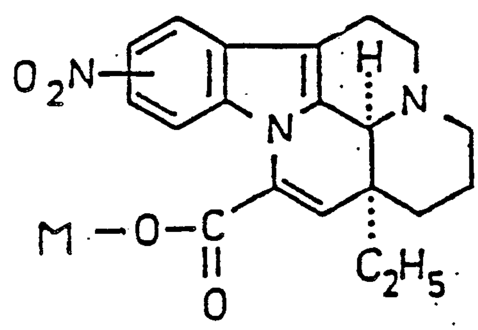

V

wherein M stands for hydrogen, a metal atom or an ammonium group or, if M is different from a metal atom, for their acid addition salts or quaternary salts, are reacted with hydroxy compounds or esters having the general formula

$$R^1—Y \qquad VI$$

in which

$R^1$ has the meanings stated in claims 1 or 3 to 5 or is an unsubstituted methyl residue and

Y stands for a hydroxy group or an acid residue, optimally in the presence of bases, or with halogenating agents and any resulting 9- and/or 11-(nitro)-apovincamic acid derivatives of the general formula I in which R stands for a halogenyl residue of the general formula II, are optionally reacted with nucleopholic reagents of the general formula

$$H—O—R^1 \qquad VII$$

or

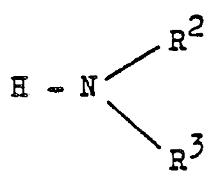

VIII

wherein $R^1$, $R^2$ and $R^3$ have the meanings stated in claim 1 or 3 to 6 or $R^1$ stands for an unsubstituted methyl residue, and any resulting 9- and/or 11-(nitro)-apovincamic acid derivatives of the general formula I in which R stands for an aminocarbonyl residue of the general formula IV, in which $R^2$ and $R^3$ are hydrogen atoms, are optionally dehydrated with dehydrating agents or

b) racemic or optically active apovincamic acid derivatives of the general formula

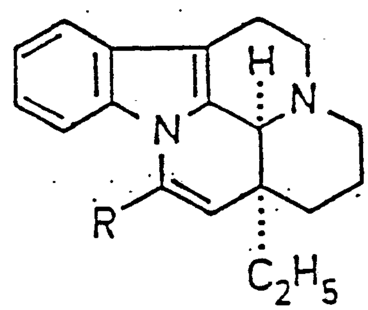

IX

wherein R has the meanings stated in claims 1 to 6, or their acid addition salts, are nitrated, and are resulting 9- and/or 11-(nitro)-apovincamic acid derivatives of the general formula I in which R stands for a hydrocarbonoxycarbonyl residue of the general formula III, in which $R^1$ is an alkyl residue having 1 or 2 carbon atom(s), or their acid addition salts are optionally transesterified or amidated and/or any resulting 9- and/or 11-(nitro)-apovincamic acid derivatives of the general formula I are optionally converted to acid addition salts or quaternary salts and/or the resulting salts of the 9- and/or 11-(nitro)-apovincamic acid derivatives of the general formula I are optionally converted to the free 9- and/or 11-(nitro)-apovincamic acid derivatives of the general formula I or to other acid addition salts and/or quaternary salts and/or the resulting racemic 9- and/or 11-(nitro)-apovincamic acid derivatives of the general formula I or their acid addition salts or quaternary salts are optionally decomposed and/or the corresponding optically active compounds are optionally racemized to form stereoisomers.

9. Medicaments, characterized by a content of 1 or more compound(s) according to claims 1 to 7 and/or 9- and/or 11-(nitro)-apovincamine as active substance(s), optionally together with 1 or more usual carrier and/or adjuvant substances.

**Claims** (for the Contracting State AT)

1. A process of producing 9- and/or 11-(nitro)-apovincamic acid derivatives of the general formula

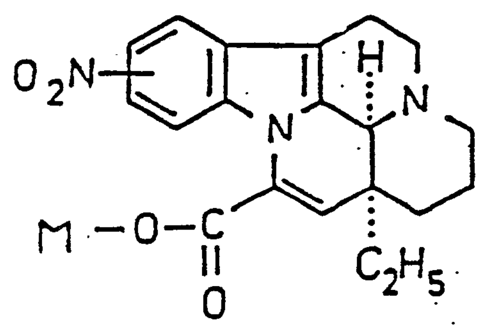

I

wherein R stands for a halogencarbonyl residue of the general formula

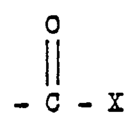

II

in which X is a halogen atom or for a hydrocarbonoxycarbonyl residue of the general formula

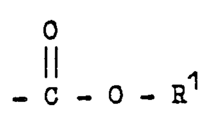

III.

in which $R^1$ is an aliphatic hydrocarbon residue having 1 to 10 carbon atom(s), or a cycloaliphatic hydrocarbon residue having 3 to 8 carbon atoms or an aromatic hydrocarbon residue having 6 to 14 carbon atoms
wherein such residue or residues may be substituted by (one) halogen atom(s), (one) primary, secondary and/or tertiary amino group(s), (one) hydroxy group(s), (one) aryl residue(s) having 6 to 14 carbon atoms, (one) alkyl, alkoxy and/or alkylthio residue(s) (each) having 1 to 8 carbon atom(s), (one) trifluoromethyl residue(s), (one) carboxyl group(s), (one) alkoxycarbonyl residue(s) having 1 to 8 carbon atom(s) in the

alkyl moiety, (one) thio, sulfinyl, sulfonyl, nitro, keto, aldehyde and/or cyan group(s) and/or (one) heteroalkyl residue(s) having one or more identical or different heteroatom(s) and optionally in addition to said members with 4 fused-on members for forming a benzene ring, which may optionally be substituted by one or more of the substituents listed or for an aminocarbonyl residue of the general formula

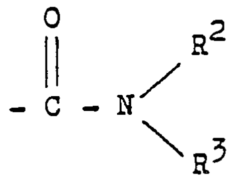

IV

wherein

$R^2$ and $R^3$ independently of each other are hydrogen and/or alkyl residues having 1 to 8 carbon atom(s), two of which may optionally be interconnected to form an alkylene residue and together with the nitrogen atom to which they are bonded constitute a pyrrolidino, piperidino, imidazolidino, piperazino or morpholino residue, or

$R^2$ is hydrogen and

$R^3$ is an amino group,

or for a cyano group and the nitro group is in porision 9 or 11, provided that

R is different from a hydrocarbonoxycarbonyl residue of the general formula III in which $R^1$ is an unsubstituted methyl residue,

and their optically active isomers and salts, characterized in that

a) racemic or optically active 9- and/or 11-(nitro)-apovincamic acids and/or derivatives thereof, which have the general formula

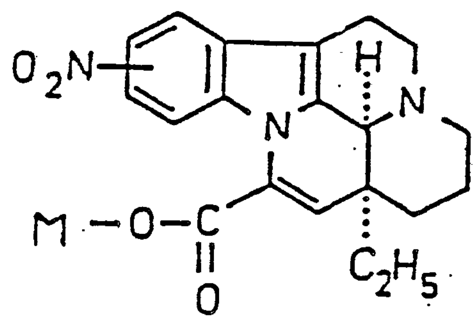

V

wherein M stands for hydrogen, a metal atom or an ammonium group or, if M is different from a metal atom, for their acid addition salts or quaternary salts, are reacted with hydroxy compounds or esters having the general formula

$$R^1—Y \qquad \text{VI}$$

in which

$R^1$ has the meanings stated above or is an unsubstituted methyl residue and

Y stands for a hydroxy group or an acid residue, optinally in the presence of bases, or with halogenating agents and any resulting 9- and/or 11-(nitro)-apovincamic acid derivatives of the general formula I in which R stands for a halogenyl residue of the general formula II, are optionally reacted with nucleopholic reagents of the general formula

$$H—O—R^1 \qquad \text{VII}$$

or

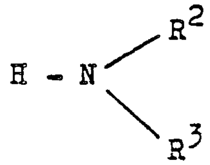

VIII

wherein $R^1$, $R^2$ and $R^3$ have the meanings stated above or $R^1$ stands for an unsubstituted methyl residue, and any resulting 9- and/or 11-(nitro)-apovincamic acid derivatives of the general formula I in which R stands for an aminocarbonyl residue of the general formula IV, in which $R^2$ and $R^3$ are hydrogen atoms, are optionally dehydrated with dehydrating agents or

b) racemic or optically active apovincamic acid derivatives of the general formula

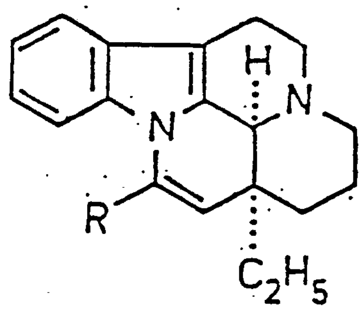

IX

wherein R has the meanings stated above, or their acid addition salts, are nitrated, and are resulting 9- and/or 11-(nitro)-apovincamic acid derivatives of the general formula I in which R stands for a hydrocarbonoxycarbonyl residue of the general formula III, in which $R^1$ is an alkyl residue having 1 or 2 carbon atom(s), or their acid addition salts are optionally transesterified or amidated and/or any resulting 9- and/or 11-(nitro)-apovincamic acid derivatives of the general formula I are optionally converted to acid addition salts or quaternary salts and/or the resulting salts of the 9- and/or 11-(nitro)-apovincamic acid derivatives of the general formula I are optionally converted to the free 9- and/or 11-(nitro)-apovincamic acid derivatives of the general formula I or to other acid addition salts and/or quaternary salts and/or the resulting racemic 9- and/or 11-(nitro)-apovincamic acid derivatives of the general formula I or their acid addition salts or quaternary salts are optionally decomposed and/or the corresponding optically active compounds are optionally racemized to form stereoisomers.

2. A process according to claim 1, characterized in that derivatives of the formula I are prepared in which the halogen atom represented by X is a chlorine or bromine atom.

3. A process according to claim 1, characterized in that derivatives of the formula I are prepared in which the aliphatic hydrocarbon residue which may be represented by $R^1$ is an alkyl, alkenyl or alkinyl residue.

4. A process according to claim 1, characterized in that derivatives of the formula I are prepared in which the cycloaliphatic hydrocarbon residue which may be represented by $R^1$ is a cycloalkyl residue or the aromatic hydrocarbon residue which may be represented by $R^1$ is monocyclic, namely the phenyl residue, or is polycyclic and uncondensed or polycyclic and condensed.

5. A process according to claim 1, 3 or 4, characterized in that derivatives of the formula I are prepared in which the substituent or substituents with which the aliphatic, cyclo-aliphatic or aromatic hydrocarbon residue which may be represented by $R^1$ may be substituted is or are (one) dialkylamino group(s) having 1 to 10 carbon atom(s) in each alkyl moiety, (one) phenyl, diphenyl or naphthyl residue(s), (one) alkyl, alkoxy and/or alkylthio residue(s) (each) having 1 to 4 carbon atom(s), (one) alkoxycarbonyl residue(s) having 1 to 4 carbon atom(s) in the alkyl moiety, (one) pyrrolyl, furyl, thienyl, pyridyl, pyranyl, pyrazolyl, imidazolyl, pyrimidinyl, indolyl and/or quinolyl residue(s).

6. A process according to claim 1, characterized in that derivatives of the formula I are prepared in which the alkyl residue(s) which may be represented by $R^2$ and/or $R^3$ has or have 1 to 4 carbon atom(s).

7. A process according to claim 1, characterized in that 9-(nitro)-apovincamic acid ethyl ester, 11-(nitro)-apovincamic acid ethyl ester, 9-(nitro)-apovincamic acid chloride, 11-(nitro)-apovincamic acid chloride, 11-[nitro]-apovincamic acid {[(dimethylamino)-n-propyl] esters}, particularly 11-[nitro]-apovincamic acid [3′-(dimethylamino)-n-propyl esters], 9-[nitro]-apovincamic acid {[(dimethylamino)-n-propyl] esters}, particularly 9-[nitro]-apovincamic acid {[3′-(dimethylamino)-n-propyl ester}, 11-(nitro)-apovincamic acid benzyl esters, 9-(nitro)-apovincamic acid benzyl esters, 11-(nitro)-apovincamic acid pyridylmethyl esters, particularly 11-(nitro)-apovincamic acid {[3′-(pyridyl)-methyl] ester}, 9-(nitro)-apovincamic acid pyridylmethyl esters, particularly 9-(nitro)-apovincamic acid {[3′-(pyridyl)-methyl] ester}, 9-(nitro)-apovincamic acid (hydroxyethyl esters), particularly 9-(nitro)-apovincamic acid {[2′-hydroxy)-ethyl ester}, 11-(nitro)-apovincamic acid (hydroxyethyl esters), particularly 11-(nitro)-apovincamic acid, {[2′-(hydroxy)-ethyl] ester}, 9-(nitro)-apovincamic acid isobutyl esters, 11-(nitro)-apovincamic acid isobutyl esters, 9-(nitro)-apovincamic acid n-octyl esters, 11-(nitro)-apovincamic acid n-octyl esters, 9-(nitro)-apovincamic acid n-propyl esters, 11-(nitro)-apovincamic acid-propyl esters, 9-(nitro)-apovincamic acid nitrile, 11-(nitro)-apovincamic acid nitrile, 9-(nitro)-apovincamic acid amide, 9-(nitro)-apovincamic acid amide, 11-(nitro)-apovincamic acid amide, 9-(nitro)-apovincamic acid diethylamide, 11-(nitro)-apovincamic acid diethylamide, 9-[nitro]-apovincamic acid [4′-(methyl)-piperidide], 11-[nitro]-apovincamic acid [4′-(methyl)-piperidide], 11-(nitro)-apovincamic acid phenyl esters, 9-(nitro)-apovincamic acid phenyl esters, 11-(nitro)-apovincamic acid hydrazide, 9-(nitro)-apovincamic acid hydrazide, 9-(nitro)-apovincamic acid propargyl ester, 11-(nitro)-apovincamic acid propargyl ester, 9-[nitro]-apovincamic acid {[2′-tri-(fluoro)-ethyl] esters}, 11-[nitro]-apovincamic acid {[2′-tri-(fluoro)-ethyl] esters}, 9-(nitro)-apovincamic acid allyl esters, 11-(nitro)-apovincamic acid allyl esters, 9-(nitro)-apovincamic acid cyclopentyl esters and 11-(nitro)-apovincamic acid cyclopentyl esters and their optically active isomers and hydrochlorides are prepared.

**Revendications** (pour les Etats contractants : BE, CH, DE, FR, GB, IT, LI, NL, SE)

1. Dérivés de l'acide 9- et/ou 11-(nitro)-apovincaminique de formule générale

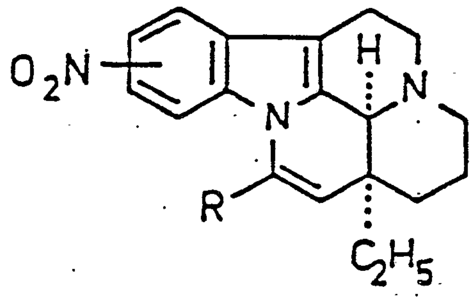

I

dans laquelle R représente un radical halogénocarbonyle de formule générale

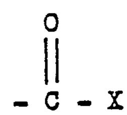

II

dans ce dernier, X désignant un atome d'halogène, ou un radical hydrocarbure-oxycarbonyle de formule générale

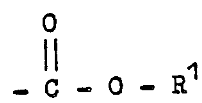

III

dans cette dernière $R^1$ désignant un radical hydrocarbure aliphatique ayant 1 à 10 atomes de carbone, un radical hydrocarbure cycloaliphatique ayant 3 à 8 atomes de carbone ou un radical hydrocarbure aromatique ayant 6 à 14 atomes de carbone, ledit ou lesdits radicaux pouvant être substitués par un ou des atomes d'halogène, un ou des groupes amino primaire, secondaire et/ou tertiaire, un ou des groupes hydroxy, un ou des radicaux aryle ayant 6 à 14 atomes de carbone, un ou des radicaux alcoyle, alcoxy et/ou alcoyl-thio ayant chacun 1 à 8 atomes de carbone, un ou des radicaux trifluorométhyle, un ou des groupes carboxyle, un ou des radicaux alcoxycarbonyle ayant 1 à 8 atomes de carbone dans la portion alcoyle, un ou des groupes thio, sulfinyle, sulfonyle, nitro, céto, aldéhyde et/ou cyano et/ou un ou des radicaux hétéroaryle présentant un ou plusieurs hétéroatomes identiques ou différents, ainsi que comportant éventuellement condensés de manière supplémentaire à leurs chaînons, 4 chaînons pour la formation d'un noyau benzène dont chacun ou plusieurs peuvent être substitués par un ou plusieurs des substituants précités, ou un radical aminocarbonyle de formule générale

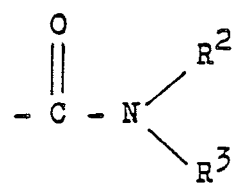

IV

dans cette dernière, $R^2$ et $R^3$ désignant indépendamment l'un de l'autre un atome d'hydrogène ou des radicaux alcoyle ayant 1 à 8 atomes de carbone, qui constituent éventuellement à raison de 2, mutuellement enchaînés sous forme de radical alcoylène conjointement avec l'atome d'azote auquel ils sont liés, un radical pyrrolidino, pipéridino, imidazolidino, pipérazino ou morpholino, ou $R^2$ désignant un atome d'hydrogène et $R^3$ représentant un groupe amino, ou un groupe cyano et le groupe nitro est en position 9 ou 11, à condition en outre que R soit différent d'un radical hydrocarbure-oxycarbonyle de formule générale III, dans laquelle $R^1$ est un radical méthyle non substitué, ainsi que leurs isomères optiquement actifs et leurs sels.

2. Dérivés de l'acide 9- et/ou 11-(nitro)-apovincaminique selon la revendication 1, caractérisés en ce que l'atome d'halogène, représenté par X, est un atome de chlore ou de brome.

3. Dérivés de l'acide 9- et/ou 11-(nitro)-apovincaminique selon la revendication 1, caractérisé en ce que le radical hydrocarbure aliphatique, que peut représenter $R^1$, est un radical alcoyle, alcényle ou

alcynyle.

4. Dérivés de l'acide 9- et/ou 11-(nitro)-apovincaminique selon la revendication 1, caractérisés en ce que le radical hydrocarbure cycloaliphatique, que peut représenter $R^1$, est un radical cycloalkyle, et que le radical hydrocarbure aromatique, que peut représenter $R^1$, est monocyclique, à savoir le radical phényle, ou polycycliquement non condensé ou polycycliquement condensé.

5. Dérivés de l'acide 9- et/ou 11-(nitro)-apovincaminique selon la revendication 1, 3 ou 4, caractérisés en ce que le ou les substituants par lequel ou lesquels peut être substitué le radical hydrocarbure aliphatique, cycloaliphatique ou aromatique, que représente $R^1$, est (ou sont) un (ou des) groupe(s) dialcoylamino ayant 1 à 10 atomes de carbone dans chaque portion alcoyle, un ou des radicaux phényle, diphényle ou naphtyle, un ou des radicaux alcoyle, alcoxy et/ou alcoylthio ayant chacun 1 à 4 atomes de carbone, un ou des radicaux alcoxycarbonyle ayant 1 à 4 atomes de carbone dans la portion alcoyle, un ou des radicaux pyrrolyle, furyle, thiényle, pyridyle, pyrannyle, pyrazolyle, imidazolyle, pyrimidinyle, indolyle et/ou quinoléyle.

6. Dérivés de l'acide 9- et/ou 11-(nitro)-apovincaminique selon la revendication 1, caractérisés en ce que le ou les radicaux alcoyle, que peuvent représenter respectivement $R^2$ et/ou $R^3$, est ou sont un ou de tels radicaux ayant 1 à 4 atomes de carbone.

7. Les dérivés de l'acide 9- et/ou 11-(nitro)-apovincaminique selon la revendication 1 : 9-(nitro)-apovincaminoate d'éthyle, 11-(nitro)-apovincaminoate d'éthyle, chlorure de 9-(nitro)-apovincaminoyle, chlorure de 11-(nitro)-apovincaminoyle, 11-(nitro)-apovincaminoates de (diméthylamino)-n-propyle, en particulier 11-(nitro)-apovincaminoate de 3'-(diméthylamino)-n-propyle, 9-(nitro)-apovincaminoates de diméthylamino-n-propyle, en particulier 9-(nitro)-apovincaminoate de 3'-(diméthylamino)-n-propyle, 11-(nitro)-apovincaminoate de benzyle, 9-(nitro)-apovincaminoate de benzyle, 11-(nitro)-apovincaminoates de pyridylméthyle, en particulier 11-(nitro)-apovincaminoate de 3'-(pyridyl)-méthyle, 9-(nitro)-apovincaminoates de pyridylméthyle, en particulier 9-(nitro)-apovincaminoate de 3'-(pyridyl)-méthyle, 9-(nitro)-apovincaminoates d'hydroxyéthyle, en particulier 9-(nitro)-apovincaminoate de 2'-(hydroxy)-éthyle, 11-(nitro)-apovincaminoates d'hydroxyéthyle, en particulier 11-(nitro)-apovincaminoate de 2'-(hydroxy)-éthyle, 9-(nitro)-apovincaminoate d'isobutyle, 11-(nitro)-apovincaminoate d'isobutyle, 9-(nitro)-apovincaminoate de n-octyle, 11-(nitro)-apovincaminoate de n-octyle, 9-(nitro)-apovincaminoate de n-propyle, 11-(nitro)-apovincaminoate de n-propyle, 9-(nitro)-apovincaminonitrile, 11-(nitro)-apovincaminonitrile, 9-(nitro)-apovincaminamide, 11-(nitro)-apovincaminamide, 9-(nitro)-apovincamino-diéthylamide, 11-(nitro)-apovincamino-diéthyl-amide, 9-(nitro)-apovincamino-4'-(méthyl)-pipéridide, 11-(nitro)-apovincamino-4'-(méthyl)-pipéridide, 11-(nitro)-apovincaminoate de phényle, 11-(nitro)-apovincaminoate de phényle, 11-(nitro)-apovincamino-hydrazide, 9-(nitro)-apovincamino-hydrazide, 9-(nitro)-apovincaminoate de propargyle, 11-(nitro)-apovincaminoate de propargyle, 9-(nitro)-apovincaminoate de 2'-tri-(fluoro)-éthyle, 11-(nitro)-apovincaminoate de 2'-tri-(fluoro)-éthyle, 9-(nitro)-apovincaminoate d'allyle, 11-(nitro)-apovincaminoate d'allyle, 9-(nitro)-apovincaminoate de cyclopentyle et 11-(nitro)-apovincaminoate de cyclopentyle ainsi que leurs isomères optiquement actifs et leurs chlorhydrates.

8. Procédé pour la préparation des dérivés de l'acide 9- et/ou 11-(nitro)-apovincaminique selon les revendications 1 à 7 et/ou de la 9- et/ou 11-(nitro)-apovincamine, caractérisé en ce que

a) on fait réagir des acides 9- et/ou 11-(nitro)-apovincaminiques racémiques ou optiquement actifs ou des dérivés de ceux-ci de formule générale

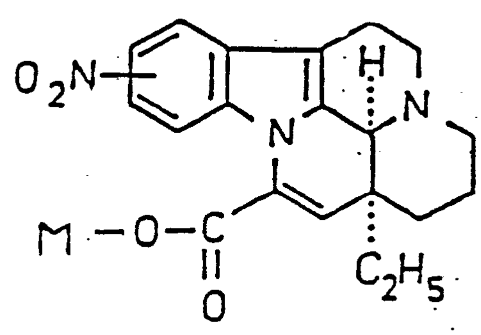

V

dans laquelle M représente un atome d'hydrogène, un atome de métal ou un groupe ammonium, ou, dans le cas où M est différent d'un atome de métal, également leurs sels d'additions d'acides ou leurs sels quaternaires, avec des composés hydroxylés ou esters de formule générale

$$R^1 — Y \qquad VI,$$

dans laquelle

$R^1$ est défini comme spécifié dans les revendications 1 ou 3 à 5 ou représente un radical méthyle non substitué et

Y représente un groupe hydroxy ou un radical acide, éventuellement en présence de bases, ou avec des agents halogénants et éventuellement on fait réagir les dérivés de l'acide 9- et/ou 11-(nitro)-apovincaminique obtenus de formule générale I, dans laquelle R représente un radical halogénocarbonyle de formule générale II, avec des réactifs nucléophiles de formule générale

$$H—O—R^1 \qquad VII$$

ou

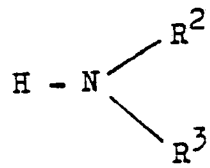

VIII

dans lesquelles $R^1$, $R^2$ et $R^3$ sont définis comme spécifié dans les revendications 1 ou 3 à 6 ou $R^1$ représente un radical méthyle non substitué, et éventuellement on soumet les dérivés de l'acide 9- et/ou 11-apovincaminiques obtenus de formule générale I, dans laquelle R représente un radical aminocarbonyle de formule générale IV, dans laquelle $R^2$ et $R^3$ désignent des atomes d'hydrogène, à une déshydratation avec des agents déshydratants ou

b) on soumet à une nitration des dérivés d'acide apovincaminique racémiques ou optiquement actifs de formule générale

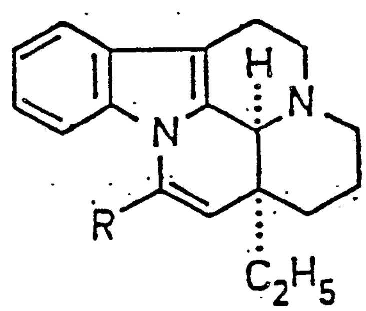

IX

dans laquelle R est défini comme spécifié dans les revendications 1 à 6, ou leurs sels d'addition d'acides, ainsi qu'éventuellement on soumet à une transestérification ou une amidation les dérivés d'acide 9- et/ou 11-(nitro)-apovincaminique obtenus de formule générale I, dans laquelle R représente un radical hydrocarbure-oxycarbonyle de formule générale III, dans laquelle $R^1$ désigne un radical alcoyle ayant 1 ou 2 atomes de carbone, ou leurs sels d'addition d'acides, et/ou éventuellement on convertit les dérivés d'acide 9- et/ou 11-(nitro)-apovincaminique obtenus de formule générale I en sels d'addition d'acides ou en sels quaternaires et/ou éventuellement on convertit les sels obtenus des dérivés de l'acide 9- et/ou 11-(nitro)-apovincaminique de formule générale I en dérivés d'acide 9- et/ou 11-(nitro)-apovincaminique libre de formule générale I ou en d'autres sels d'addition d'acides ou sels quaternaires et/ou éventuellement on effectue un dédoublement des dérivés d'acide 9- et/ou 11-(nitro)-apovincaminique racémiques obtenus de formule générale I ou de leurs sels d'addition d'acides ou sels quaternaires ou on effectue une racémisation des composés optiquement actifs correspondants en stéréo-isomères.

9. Médicaments, caractérisés par une teneur en un ou plusieurs composés selon les revendications 1 à 7 et/ou en 9- et/ou 11-(nitro)-apovincamine en tant que substance(s) active(s), d'une manière convenable conjointement avec un ou plusieurs véhicules et/ou adjuvants courants.

**Revendications** (pour l'Etat contractant AT)

1. Procédé pour la préparation des dérivés de l'acide 9- et/ou 11-(nitro)-apovincaminique de formule générale

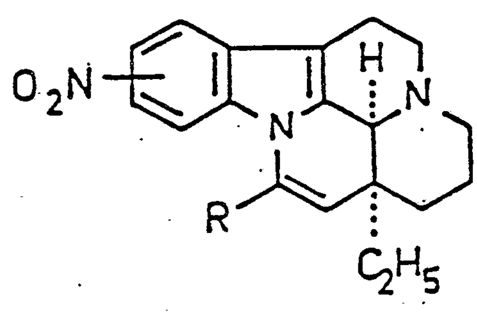

I

dans laquelle R représente un radical halogénocarbonyle de formule générale

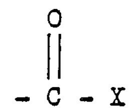

.II

dans ce dernier, X désignant un atome d'halogène, ou un radical hydrocarbure-oxycarbonyle de formule générale

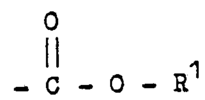

III

dans cette dernière $R^1$ désignant un radical hydrocarbure aliphatique ayant 1 à 10 atomes de carbone, un radical hydrocarbure cycloaliphatique ayant 3 à 8 atomes de carbone ou un radical hydrocarbure atomatique ayant 6 à 14 atomes de carbone, ledit ou lesdits radicaux pouvant être substitués par un ou des atomes d'halogène, un ou des groupes amino primaire, secondaire et/ou tertiaire, un ou des groupes hydroxy, un ou des radicaux aryle ayant 6 à 14 atomes de carbone, un ou des radicaux alcoyle, alcoxy et/ou alcoyl-thio ayant chacun 1 à 8 atomes de carbone, un ou des radicaux trifluorométhyle, un ou des groupes carboxyle, un ou des radicaux alcoxycarbonyle ayant 1 à 8 atomes de carbone dans la portion alcoyle, un ou des groupes thio, sulfinyle, sulfonyle, nitro, céto, aldéhyde et/ou cyano et/ou un ou des radicaux hétéroaryle présentant un ou plusieurs hétéroatomes identiques ou différents, ainsi que comportant éventuellement condensés de manière supplémentaire à leurs chaînons, 4 chaînons pour la formation d'un noyau benzène dont chacun ou plusieurs peuvent être substitués par un ou plusieurs des substituants précités, ou un radical aminocarbonyle de formule générale

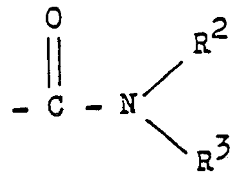

.IV

dans cette dernière, $R^2$ et $R^3$ désignant indépendamment l'un de l'autre un atome d'hydrogène ou des radicaux alcoyle ayant 1 à 8 atomes de carbone, qui constituent éventuellement à raison de 2, mutuellement enchaînés sous forme de radical alcoylène conjointement avec l'atome d'azote auquel ils sont liés, un radical pyrrolidino, pipéridino, imidazolidino, pipérazino ou morpholino, ou $R^2$ désignant un atome d'hydrogène et $R^3$ représentant un groupe amino, ou un groupe cyano et le groupe nitro est en position 9 ou 11, à condition en outre que R soit différent d'un radical hydrocarbure-oxycarbonyle de formule générale III, dans laquelle $R^1$ est un radical méthyle non substitué, ainsi que leurs isomères optiquement actifs et leurs sels caractérisé en ce que

a) on fait réagir des acides 9- et/ou 11-(nitro)-apovincaminiques racémiques ou optiquement actifs ou des dérivés de ceux-ci de formule générale

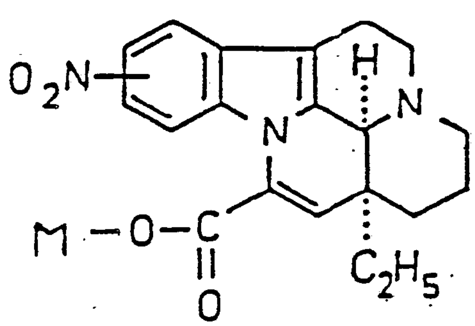

.V

dans laquelle M représente un atome d'hydrogène, un atome de métal ou un groupe ammonium, ou, dans le cas où M est différent d'un atome de métal, également leurs sels d'additions d'acides ou leurs sels quaternaires, avec des composés hydroxylés ou esters de formule générale

$R^1 — Y$ VI,

dans laquelle

$R^1$ est défini comme spécifié ci-dessus
ou représente un radical méthyle non substitué et
Y représente un groupe hydroxy ou un radical acide,
éventuellement en présence de bases, ou avec des agents halogénants et éventuellement on fait réagir les dérivés de l'acide 9- et/ou 11-(nitro)-apovincaminique obtenus de formule générale I, dans laquelle R représente un radical halogénocarbonyle de formule générale II, avec des réactifs nucléophiles de formule générale

H—O—$R^1$ VII

ou

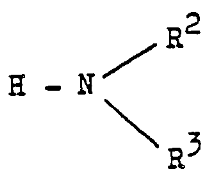

VIII

dans lesquelles $R^1$, $R^2$ et $R^3$ sont définis comme spécifié ci-dessus ou $R^1$ représente un radical méthyle non substitué, et éventuellement on soumet les dérivés de l'acide 9- et/ou 11-apovincaminiques obtenus de formule générale I, dans laquelle R représente un radical aminocarbonyle de formule générale IV, dans laquelle $R^2$ et $R^3$ désignent des atomes d'hydrogène, à une déshydratation avec des agents déshydratants ou

b) on soumet à une nitration des dérivés d'acide apovincaminique racémiques ou optiquement actifs de formule générale

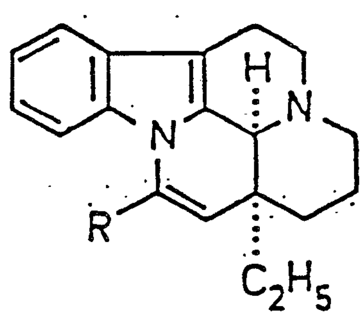

IX

dans laquelle R est défini comme spécifié ci-dessus ou leurs sels d'addition d'acides, ainsi qu'éventuellement on soumet à une transestérification ou une amidation les dérivés d'acide 9- et/ou 11-(nitro)-apovincaminique obtenus de formule générale I, dans laquelle R représente un radical hydrocarbure-oxycarbonyle de formule générale III, dans laquelle $R^1$ désigne un radical alcoyle ayant 1 ou 2 atomes de carbone, ou leurs sels d'addition d'acides, et/ou éventuellement on convertit les dérivés d'acide 9- et/ou 11-(nitro)-apovincaminique obtenus de formule générale I en sels d'addition d'acides ou en sels quaternaires et/ou éventuellement on convertit les sels obtenus des dérivés de l'acide 9- et/ou 11-(nitro)-apovincaminique de formule générale I en degrés d'acide 9- et/ou 11-(nitro)-apovincaminique libre de formule générale I ou en d'autres sels d'addition d'acides ou sels quaternaires et/ou éventuellement on effectue un dédoublement des dérivés d'acide 9- et/ou 11-(nitro)-apovincaminique racémiques obtenus de formule générale I ou de leurs sels d'addition d'acides ou sels quaternaires ou on effectue une racémisation des composés optiquement actifs correspondants en stéréo-isomères.

2. Procédé selon la revendication 1, caractérisé en ce que les dérivés de formule générale I sont préparés, dans laquelle l'atome d'halogène, représenté par X, est un atome de chlore ou de brome.

3. Procédé selon la revendication 1, caractérisé en ce que les dérivés de formule générale I sont préparés, dans laquelle le radical hydrocarbure aliphatic, que peut représenter $R^1$, est un radical alcoyle, alcényle ou alcynyle.

4. Procédé selon la revendication 1, caractérisé en ce que les dérivés de formule générale I sont préparés, dans laquelle le radical hydrocarbure cycloaliphatique, que peut représenter $R^1$, est un radical cycloalkyle, et que le radical hydrocarbure aromatique, que peut représenter $R^1$, est monocyclique, à savoir le radical phényle, ou polycycliquement non condensé ou polycycliquement condensé.

5. Procédé selon la revendication 1, 3 ou 4, caractérisé en ce que les dérivés de formule I sont préparés, dans laquelle le ou les substituants par lequel ou lesquels peut être substitué le radical hydrocarbure aliphatique, cycloaliphatique ou aromatique, que représente $R^1$, est (ou sont) un (ou des) groupe(s) dialccylamino ayant 1 à 10 atomes de carbone dans chaque portion alcoyle, un ou des radicaux phényle, diphényle ou naphtyle, un ou des radicaux alcoyle, alcoxy et/ou alcoylthio ayant chacun 1 à 4 atomes de carbone, un ou des radicaux alcoxycarbonyle ayant 1 à 4 atomes de carbone dans la portion alcoyle, un ou des radicaux pyrrolyle, furyle, thiényle, pyridyle, pyrannyle, pyrazolyle, imidazolyle, pyrimidinyle, indolyle et/ou quinoléyle.

6. Procédé selon revendication 1, caractérisé en ce que les dérivés de formule I sont préparés, dans laquelle le/ou les radicaux alcoyle, que peuvent représenter respectivement $R^2$ et/ou $R^3$, est/ou sont un ou de tels radicaux ayant 1 à 4 atomes de carbone.

7. Procédé selon revendication 1, caractérisé en ce que 9-(nitro)-apovincaminoate d'éthyle, 11-(nitro)-apovincaminoate d'éthyle, chlorure de 9-(nitro)-apovincaminoyle, chlorure de 11-(nitro)-apovincaminoyle, 11-(nitro)-apovincaminoates de (diméthylamino)-n-propyle, en particulier 11-(nitro)-apovincaminoate de 3'-(diméthylamino)-n-propyle, 9-(nitro)-apovincaminoates de diméthylamino-n-propyle, en particulier 9-(nitro)-apovincaminoate de 3'-(diméthylamino)-n-propyle, 11-(nitro)-apovincaminoate de benzyle, 9-(nitro)-apovincaminoate de benzyle, 11-(nitro)-apovincaminoates de pyridylméthyle, en particulier 11-(nitro)-apovincaminoate de 3'-(pyridyl)-méthyle, 9-(nitro)-apovincaminoates de pyridylméthyle, en particulier 9-(nitro)-apovincaminoate de 3'-(pyridyl)-méthyle, 9-(nitro)-apovincaminoates d'hydroxyéthyle, en particulier 9-(nitro)-apovincaminoate de 2'-(hydroxy)-éthyle, 11-(nitro)-apovincaminoates d'hydroxyéthyle, en particulier 11-(nitro)-apovincaminoate de 2'-(hydroxy)-éthyle, 9-(nitro)-apovincaminoate d'isobutyle, 11-(nitro)-apovincaminoate d'isobutyle, 9-(nitro)-apovincaminoate de n-octyle, 11-(nitro)-apovincaminoate de n-octyle, 9-(nitro)-apovincaminoate de n-propyle, 11-(nitro)-apovincaminoate de n-propyle, 9-(nitro)-apovincaminonitrile, 11-(nitro)-apovincaminonitrile, 9-(nitro)-apovincaminamide, 11-(nitro)-apovincaminamide, 9-(nitro)-apovincamino-diéthylamide, 11-(nitro)-apovincamino-diéthylamide, 9-(nitro)-apovincamino-4'-(méthyl)-pipéridide, 11-(nitro)-apovincamino-4'-(méthyl)-pipéridide, 11-(nitro)-apovincaminoate de phényle, 11-(nitro)-apovincaminoate de phényle, 11-(nitro)-apovincamino-hydrazide, 9-(nitro)-apovincamino-hydrazide, 9-(nitro)-apovincaminoate de propargyle, 11-(nitro)-apovincaminoate de propargyle, 9-(nitro)-apovincaminoate de 2'-tri-(fluoro)-éthyle, 11-(nitro)-apovincaminoate de 2'-tri-(fluoro)-éthyle, 9-(nitro)-apovincaminoate d'allyle, 11-(nitro)-apovincaminoate d'allyle, 9-(nitro)-apovincaminoate de cyclopentyle et 11-(nitro)-apovincaminoate de cyclopentyle ainsi que leurs isomères optiquement actifs et leurs chlorhydrates sont préparés.